(19) Europäisches Patentamt
European Patent Office
Office européen des brevets

(11) Veröffentlichungsnummer : **0 124 479**
**B1**

(12) # EUROPÄISCHE PATENTSCHRIFT

(45) Veröffentlichungstag der Patentschrift : **20.01.88**

(51) Int. Cl.⁴ : **C 07 D233/46**, A 61 K 31/415

(21) Anmeldenummer : **84810155.6**

(22) Anmeldetag : **30.03.84**

(54) **Iminosulfonamide und Verfahren zu ihrer Herstellung, die pharmazeutische Präparate solche Verbindungen enthalten, sowie die Verbindungen zur Verwendung.**

(30) Priorität : **05.04.83 CH 1817/83**

(43) Veröffentlichungstag der Anmeldung : **07.11.84 Patentblatt 84/45**

(45) Bekanntmachung des Hinweises auf die Patenterteilung : **20.01.88 Patentblatt 88/03**

(84) Benannte Vertragsstaaten : **AT BE CH DE FR GB IT LI LU NL SE**

(56) Entgegenhaltungen : **DE-B- 1 912 847**
**Die Akte enthält technische Angaben, die nach dem Eingang der Anmeldung eingereicht wurden und die nicht in dieser Patentschrift enthalten sind.**

(73) Patentinhaber : **CIBA-GEIGY AG**
**Klybeckstrasse 141**
**CH-4002 Basel (CH)**

(72) Erfinder : **Schweizer, Ernst, Dr.**
**Hollenweg 59**
**CH-4144 Arlesheim (CH)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft Iminosulfonamide und Verfahren zu ihrer Herstellung, pharmazeutische Präparate die solche Iminosulfonamide enthalten, sowie die Iminosulfonamide zur Verwendung als pharmakologisch aktive Verbindungen.

In der DE-C-1 912 847 werden im Imidazolidinrest substituierte 1-(p-Acylamidoäthylbenzolsulfonyl)-2-imino-imidazolidine beschrieben, doch werden die erfindungsgemässen Verbindungen weder als Gruppen noch einzeln offenbart. Im nach veröffentlichten Journal of Medicinal Chemistry, 1983, 26, Vol. 7, (Juli) 964-970 wird an Hand von ausgeführten Vergleichsversuchen gezeigt, dass die erfindungsgemäss herstellbaren Verbindungen den nächstvergleichbaren Verbindungen an hypoglykämischer Wirksamkeit überraschenderweise weit überlegen sind.

Die Erfindung betrifft 1-{4-[2-(Acylamino)-äthyl]-phenylsulfonyl}-3-cyclohexyl-2-imino-imidazolidin-Verbindungen der Formel (I)

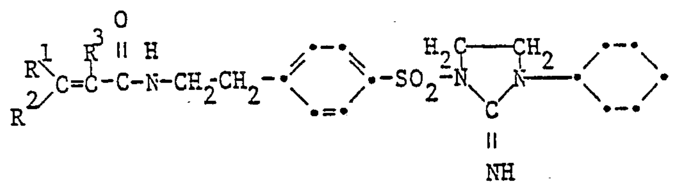

(I)

worin $R^1$ für $C_{1-3}$-Alkyl und $R^2$ und $R^3$ unabhängig voneinander für Wasserstoff oder $C_{1-3}$-Alkyl stehen, und Salze von solchen Verbindungen. $C_{1-3}$-Alkylgruppen $R^2$ bzw. $R^3$ bedeuten in erster Linie Methyl, während $R^1$ z. B. für Methyl, Aethyl, n-Propyl oder Isopropyl stehen kann.

Die Verbindungen der Formel I können mit Bezug auf $R^1$ und die Carbonylgruppe die cis- und vorzugsweise trans-Konfiguration aufweisen.

Die neuen Verbindungen können in Form von Säureadditionssalzen, in erster Linie von pharmazeutisch verwendbaren, nicht-toxischen Säureadditionssalze vorliegen. Geeignete Salze sind z. B. solche mit anorganischen Säuren, wie Chlorwasserstoffsäure, Bromwasserstoffsäure, Schwefelsäure oder Phosphorsäure, oder mit organischen Säuren, wie aliphatischen, cycloaliphatischen, aromatischen oder heterocyclischen Carbon- oder Sulfonsäuren, z. B. Ameisen-, Essig-, Propion-, Bernstein-, Glykol-, Milch-, Aepfel-, Wein-, Zitronen-, Malein-, Hydroxymalein-, Brenztrauben-, Fumar-, Benzoe-, 4-Aminobenzoe-, Anthranil-, 4-Hydroxybenzoe-, Salicyl-, Embon-, Methansulfon-, Aethansulfon-, 2-Hydroxy-äthansulfon-, Aethylensulfon-, Toluolsulfon-, Naphthalinsulfon- oder Sulfanilsäure, oder mit anderen sauren organischen Verbindungen, wie Ascorbinsäure.

Die vorliegende Erfindung betrifft in erster Linie Verbindungen der Formel (I), worin $R^1$ für $C_{1-3}$-Alkyl, $R^2$ vorzugsweise für Wasserstoff, ferner für Methyl, und $R^3$ für Wasserstoff oder Methyl stehen, wobei diese Verbindungen mit Bezug auf den Rest $R^1$ und die Carbonylgruppe die cis- und vorzugsweise die trans-Konfiguration aufweisen können, und insbesondere das 3-Cyclohexyl-2-imino-1-{4-[2-(crotonylamino)-äthyl]-phenylsulfonyl}-imidazolidin (trans), und Salze, insbesondere pharmazeutisch verwendbare Salze davon.

Die neuen Verbindungen der vorliegenden Erfindung kann man in an sich bekannter Weise erhalten, z. B. (a) indem man eine Verbindung der Formel (II)

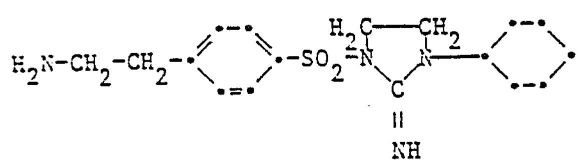

(II)

oder ein Derivat davon mit einer Säure der Formel (III)

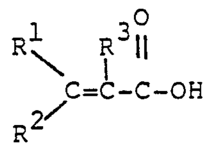

oder einem reaktionsfähigen Derivat davon umsetzt, und, wenn erwünscht, ein erfindungsgemäss erhältliches Salz in die freie Verbindung oder in ein anderes Salz, und/oder eine erfindungsgemäss erhältliche freie Verbindung in ein Salz umwandelt und/oder, wenn erwünscht, ein Isomerengemisch in die einzelnen Isomeren auftrennt.

Ein Derivat des Ausgangsmaterials der Formel (II) kann z. B. ein Silylderivat, wie ein Triniederalkyl-,

wie Trimethylsilylderivat, ferner auch ein Säureadditionssalz, wie das Salz mit einer der vorgenannten Säuren sein.

Ein reaktionsfähiges Derivat einer Säure der Formel (III) ist in erster Linie ein Anhydrid, inkl. ein asymmetrisches Anhydrid, wie u. a. das Anhydrid mit einer starken anorganischen Säure, wie einer Halogenwasserstoffsäure, insbesondere das entsprechende Säurechlorid, oder mit einem Kohlensäurehalbester, wie einem Kohlensäure-niederalkyl-, z. B. Isopropyl-halbester, oder ein inneres Anhydrid, d. h. die entsprechende Ketenverbindung. Geeignete reaktionsfähige Säurederivate sind ebenfalls Ester, wie ein Niederalkyl-, z. B. Methyl- oder Aethylester, insbesondere jedoch aktivierte Ester von Säuren der Formel (III), wie geeignet substituierte Phenyl-, z. B. 4-Nitrophenyl- oder Pentachlorphenyl-, oder Methyl-, z. B. Cyanmethylester.

Die Reaktion kann in an sich bekannter Weise durchgeführt werden, wenn notwendig, in Gegenwart eines Kondensationsmittels, z. B. eines Carbodiimids, wie N,N'-Dicyclohexyl-carbodiimid, wobei solche in erster Linie bei Verwendung einer freien Säure der Formel (III) zur Anwendung kommen, oder eines säurebindenden Mittels, wie z. B. einer anorganischen Base oder Salzes, wie eines Alkalimetallhydroxids, Alkalimetallhydrogencarbonats, Alkalimetallcarbonats oder Alkalimetallphosphats, wie der entsprechenden Natrium- oder Kaliumverbindung. Ferner kann auch eine organische Base, z. B. Pyridin, Trimethyl- oder Triäthylamin, N,N-Diisopropyl-äthylamin oder Collidin, verwendet werden, die, im Ueberschuss zugefügt, gleichzeitig auch als Lösungsmittel eingesetzt werden kann. Ueblicherweise arbeitet man in Gegenwart eines Lösungsmittels, vorzugsweise eines gegebenenfalls mit Wasser mischbaren, inerten organischen Lösungsmittels in An- oder Abwesenheit von Wasser. Geeignete inerte organische Lösungsmittel sind z. B. Kohlenwasserstoffe, wie Benzol, Toluol oder Xylol, Aether, wie Diäthyläther, Dioxan oder Tetrahydrofuran, halogenierte Kohlenwasserstoffe, wie Methylenchlorid, oder niedere Ketone, wie Aceton oder Methyläthylketon. Die Reaktion kann, wenn notwendig, unter Kühlen oder Erwärmen, unter erhöhtem Druck und/oder in einer Inertgas-, wie Stickstoffatmosphäre durchgeführt werden.

Die Ausgangsstoffe der Formeln (II) und (III) sind bekannt ; diejenigen der Formel (II) kann man z. B. durch Behandeln eines Säureadditionssalzes, wie des Hydrochlorids von 4-(2-Amino-alkyl)-phenylsulfonamid mit N-(2-Chlor-äthyl)-N-cyclohexyl-cyanamid in Gegenwart eines Alkalimetall-, z. B. Kaliumhydroxids erhalten.

Die neuen Verbindungen der Formel (I) können ebenfalls erhalten werden, wenn man (b) ein reaktionsfähiges Derivat einer Sulfonsäure der Formel (IV)

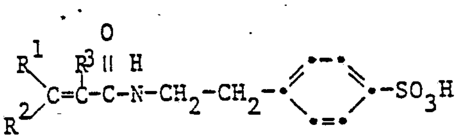

(IV)

mit einer Verbindung der Formel (V)

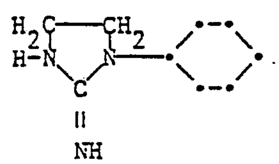

(V)

oder einem Derivat davon umsetzt, und, wenn erwünscht, die zusätzlichen Verfahrensschritte durchführt.

Reaktionsfähige Derivate von Sulfonsäuren der Formel (IV) sind in erster Linie deren Anhydride, insbesondere gemischte Anhydride mit starken Säuren, wie Halogenwasserstoffsäuren, insbesondere die entsprechenden Chloride, ferner auch die entsprechenden symmetrischen Anhydride.

Ein Derivat einer Verbindung der Formel (V) kann z. B. ein Silylderivat, wie ein Triniederalkyl-, wie Trimethylsilylderivat, ferner auch ein Säureadditionssalz, wie das Salz mit einer der vorgenannten Säuren sein.

Die Reaktion kann in an sich bekannter Weise durchgeführt werden, wenn notwendig in Gegenwart eines säurebindenden Mittels, wie z. B. einer anorganischen Base oder Salzes, wie eines Alkalimetallhydroxids, Alkalimetallhydrogencarbonats, Alkalimetallcarbonats oder Alkalimetallphosphats, wie der entsprechenden Natrium- oder Kaliumverbindung. Ferner kann auch eine organische Base, z. B. Pyridin, Trimethyl- oder Triäthylamin, N,N-Diisopropyl-äthylamin oder Collidin, verwendet werden, die, im Ueberschuss zugefügt, gleichzeitig auch als Lösungsmittel eingesetzt werden können. Ueblicherweise arbeitet man in Gegenwart eines Lösungsmittels, vorzugsweise eines gegebenenfalls mit Wasser mischbaren, inerten organischen Lösungsmittels in An- oder Abwesenheit von Wasser. Geeignete inerte organische Lösungsmittel sind z. B. Kohlenwasserstoffe, wie Benzol, Toluol oder Xylol, Aether, wie Diäthyläther, Dioxan oder Tetrahydrofuran, halogenierte Kohlenwasserstoffe, wie Methylenchlorid, oder niedere Ketone, wie Aceton oder Methyläthylketon. Die Reaktion kann, wenn notwendig, unter Kühlen oder Erwärmen, unter erhöhtem Druck und/oder in einer Inertgas-, wie Stickstoffatmosphäre durchgeführt werden.

Die Ausgangsstoffe sind bekannt oder können in an sich bekannter Weise hergestellt werden, wobei man z. B. Halogenide von Säureverbindungen der Formel (IV) z. B. durch Behandeln eines entsprechend substituierten Phenyläthylamins mit einer Halogen-, insbesondere Chlorsulfonsäure erhalten kann.

Die neuen Verbindungen der Formel (I) können ebenfalls erhalten werden, indem man (c) eine Verbindung der Formel (VI)

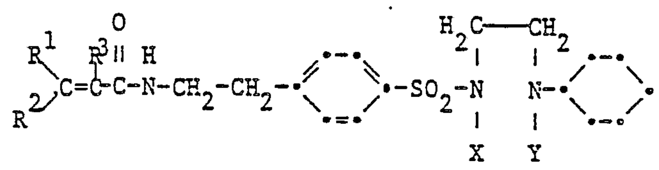

(VI)

worin einer der Reste X und Y Cyan und der andere Wasserstoff bedeuten, ringschliesst, und, wenn erwünscht, die zusätzlichen Verfahrensschritte durchführt.

Die obige Cyclisierung kann unter an sich bekannten Reaktionsbedingungen durchgeführt werden. Dabei wird üblicherweise das Ausgangsmaterial der Formel (VI) in situ gebildet und, ohne isoliert zu werden, direkt in eine Verbindung der Formel (I) übergeführt.

So kann man z. B. (ca) eine Verbindung der Formel (VIa)

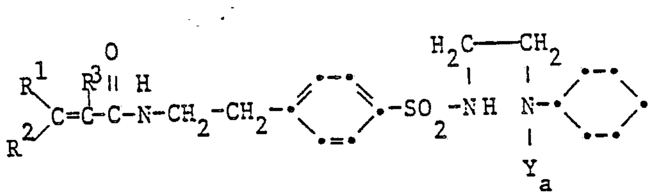

(VIa)

worin $Y_a$ für Wasserstoff oder einen unter den Reaktionsbedingungen abspaltbaren Rest steht, mit einem reaktionsfähigen Derivat der Cyansäure umsetzen ; man kann in dieser Weise direkt eine Verbindung der Formel (I) erhalten ohne ein Zwischenprodukt der Formel (VI) zu isolieren.

In einem Ausgangsmaterial der Formel (VIa) ist z. B. ein unter den Reaktionsbedingungen, d. h. beim Umsetzen mit dem Cyansäurederivat, abspaltbarer Rest eine gegebenenfalls, z. B. durch eine Arylgruppe, wie gegebenenfalls, z. B. durch Niederalkyl, wie Methyl, Niederalkoxy, wie Methoxy, oder Halogen, wie Chlor, substituiertes Phenyl, mono-, di- oder trisubstituierte oder durch, am Verknüpfungskohlenstoffatom ungesättigtes Niederalkenyl, wie Vinyl, substituierte Methylgruppe.

Ein reaktionsfähiges Derivat der Cyansäure ist z. B. ein Halogenid, wie Chlorcyan oder Bromcyan, oder ein Ester, wie ein Niederalkyl- oder besonders ein Phenylester davon.

Die Reaktion kann in an sich bekannter Weise durchgeführt werden, wenn notwendig, in Anwesenheit eines säurebindenden Mittels, wie einer anorganischen Base, z. B. eines Alkalimetallhydroxids, -hydrogencarbonats, -carbonats oder -phosphats, wie der entsprechenden Natrium- oder Kaliumverbindung. Ferner können auch Calciumcarbonat, sowie Calciumphosphat oder Magnesiumcarbonat eingesetzt werden.

Die Umsetzung erfolgt in Abwesenheit, vorzugsweise jedoch in Anwesenheit eines inerten, üblicherweise organischen Lösungsmittels, wenn erwünscht, in Gegenwart von Wasser. Geeignete Lösungsmittel sind beispielsweise Kohlenwasserstoffe, wie Benzol, Toluol oder Xylol, Niederalkanole, wie Methanol oder Aethanol, Aether, wie Diäthyläther, Dioxan oder Tetrahydrofuran, halogenierte Kohlenwasserstoffe, wie Methylenchlorid, niedere Ketone, wie Aceton oder Methyläthylketon, Carbonsäureester, wie Essigsäureäthylester, Carbonsäurenitrile, wie Acetonitril, oder Sulfone, wie Tetrahydro-thiophen-1,1-dioxid. Dabei arbeitet man, wenn notwendig, unter Kühlen oder Erwärmen, unter erhöhtem Druck und/oder in einer Inertgas-, wie Stickstoffatmosphäre.

Ein Ausgangsmaterial der Formel (VIa) kann z. B. durch Behandeln eines

4-[2-( 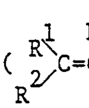-Carbonylamino)-äthyl]-benzolsulfonylchlorids

mit Aziridin und Umsetzen des so erhältlichen

N-{4-[2-( 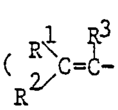-Carbonylamino)-äthyl]-phenylsulfonyl}-aziridins

mit einem N-$Y_a$-Cyclohexylamin erhalten werden.

Ferner kann man (cb) eine Verbindung der Formel (VIb)

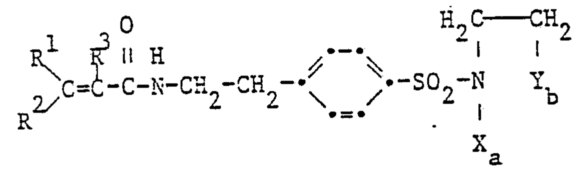

(VIb)

worin $X_a$ Wasserstoff und $Y_b$ eine reaktionsfähige veresterte Hydroxygruppe bedeuten, oder worin $X_a$ und $Y_b$ zusammen eine Bindung bilden, mit N-Cyclohexyl-cyanamid oder einem Derivat davon umsetzen ; man kann so direkt Verbindungen der Formel (I) erhalten, ohne ein Zwischenprodukt der Formel (VI) zu isolieren.

Eine reaktionsfähige veresterte Hydroxygruppe $Y_b$ ist mit einer starken anorganischen oder organischen Säure, wie einer Halogen-, z. B. Chlor- oder Bromwasserstoffsäure, oder einer organischen Sulfonsäure, wie p-Toluol- oder Methansulfonsäure, verestert.

Eine Derivat des N-Cyclohexyl-cyanamids ist in erster Linie ein Metall-, wie Alkali-, z. B. Lithium-, Natrium- oder Kalium-, oder Erdalkalimetall-, z. B. Calciumderivat. Ein solches wird in erster Linie bei der Reaktion mit einem Ausgangsmaterial der Formel (IIb) eingesetzt, worin $X_a$ und $Y_b$ zusammen eine Bindung bilden.

Die Reaktion kann in an sich bekannter Weise durchgeführt werden, wobei man bei Verwendung von Ausgangsstoffen der Formel (VIb), worin $X_a$ für Wasserstoff und $Y_b$ für eine reaktionsfähige veresterte Hydroxygruppe stehen, vorzugsweise in Gegenwart von säurebindenden Mitteln, wie anorganischen oder organischen Basen, z. B. Alkali- oder Erdalkalimetallhydroxiden, wie Natrium- oder Kaliumhydroxid, arbeitet. Wenn notwendig oder erwünscht, wird die Reaktion in Gegenwart eines Lösungsmittels, wie eines Aethers, z. B. Diäthyläther, Tetrahydrofuran, Dioxan, Anisol oder Aethylenglycoldimethyläther, ferner eines Niederalkanols, z. B. Butanol, eines Carbonsäureamids, wie Dimethylformamid, oder eines Sulfoxids, wie Dimethylsulfoxid, unter Kühlen oder Erwärmen, in einem geschlossenen Gefäss und/oder in einer Inertgas-, wie Stickstoffatmosphäre durchgeführt.

Die Ausgangsstoffe der Formel (VIb) können z. B. erhalten werden, wenn man ein

4-[2-( 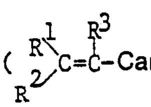 -Carbonylamino)-äthyl]-benzolsulfonylchlorid

mit Arizidin oder mit einem Säureadditionssalz eines 2-$Y_b$-Aethylamins, worin $Y_b$ für eine reaktionsfähige veresterte Hydroxygruppe steht, vorzugsweise in Gegenwart einer Base, wie eines Alkalimetall-, z. B. Natrium- oder Kaliumhydroxids, umsetzt.

Ferner kann man (cc) eine Verbindung der Formel (VIc)

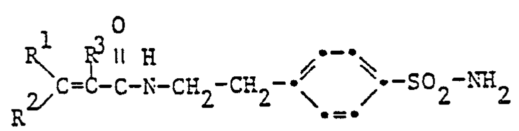

(VIc)

mit einem reaktionsfähigen veresterten N-Cyclohexyl-N-(2-hydroxyäthyl)-cyanamid oder einem Derivat davon umsetzen ; man kann so direkt Verbindungen der Formel (I) erhalten ohne ein Zwischenprodukt der Formel (VI) zu isolieren.

In einem rekationsfähigen Ester von N-(2-Hydroxy-äthyl)-cyanamid ist die Hydroxygruppe mit einer starken anorganischen oder organischen Säure, wie einer Halogen-, z. B. Chlor- oder Bromwasserstoffsäure, oder einer organischen Sulfonsäure, wie p-Toluol- oder Methansulfonsäure verestert.

Ein Derivat des Cyanamid-Ausgangsmaterials ist z. B. ein Säureadditionssalz, z. B. mit einer Mineral-, wie Halogen-, z. B. Chlorwasserstoffsäure.

Die Reaktion wird in an sich bekannter Weise durchgeführt, vorteilhafterweise in Gegenwart eines säurebindenden Mittels, wie einer anorganischen Base, z. B. eines Alkalimetall-, wie Natrium- oder Kaliumhydroxides, -hydrogencarbonates, -carbonates und -phosphates, ferner auch einer organischen Base, wie eines tertiären Amins, z. B. N,N-Diisopropyläthylamin. Wenn notwendig oder erwünscht, arbeitet man in Anwesenheit eines geeigneten Lösungsmittels, gegebenenfalls in Gegenwart von Wasser, wie eines Niederalkanols, z. B. Butanol, Aethers, z. B. Dioxan oder Diäthylenglycol-monomethyläther, Carbonsäureamids, z. B. N,N-Dimethylformamid, oder Sulfoxids, z. B. Dimethylsulfoxid, unter Kühlen oder Erwärmen, in einem geschlossenen Gefäss und/oder in einer Inertgas-, wie Stickstoffatmosphäre.

Die Ausgangsstoffe der Formel (VIc) können in an sich bekannter Weise, z. B. durch Aminolyse eines

4-[2-( 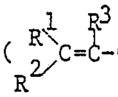 -Carbonylamino)-äthyl]-benzolsulfonylchlorids bzw. durch Behandeln von 1-Cyclohexyl-aziridin

mit einem chlorids bzw. durch Behandeln von 1-Cyclohexyl-aziridin mit einem reaktionsfähigen Derivat der Cyansäure, wie einem Halogen-, z. B. Bromcyan, erhalten werden.

Eine weitere Verfahrensvariante besteht darin, dass man (cd) eine Additionssalz-Verbindung der Formel (VId)

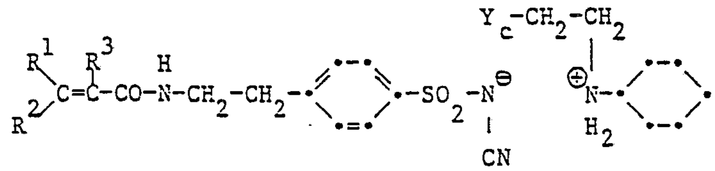

(VId)

worin $Y_c$ für eine reaktionsfähige veresterte Hydroxygruppe steht, cyclisiert ; man kann so direkt Verbindungen der Formel (I) erhalten ohne ein Zwischenprodukt der formel (VI) zu isolieren.

Eine reaktionsfähige veresterte Hydroxygruppe $Y_c$ ist in erster Linie Halogen, insbesondere Brom, ferner Chlor, kann jedoch auch eine organische Sulfonyloxygruppe, wie Methylsulfonyloxy oder 4-Methylphenylsulfonyloxy sein.

Die Reaktion wird in an sich bekannter Weise durchgeführt, vorzugsweise unter Erwärmen und, wenn notwendig oder erwünscht, in Gegenwart eines, in erster Linie hochsiedenden Lösungsmittels, wie eines Aethers, z. B. Diäthylenglycol-dimethyläther, oder eines Carbonsäureamids, z. B. N,N-Dimethylformamid, in einem geschlossenen Gefäss und/oder in einer Inertgas-, z. B. Stickstoffatmosphäre.

Das Ausgangsmaterial der Formel (VId) kann man z. B. erhalten, wenn man ein

4-[2-( 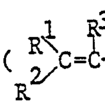 -Carbonylamino)-äthyl]-benzolsulfonylchlorid z. B. durch Behandeln

mit Dinatriumcyanamid in Wasser zu einem Natriumderivat des entsprechenden N-Cyanbenzolsulfonamid umsetzt und dieses mit einem N-(2-$Y_c$-Aethyl)-cyclohexylamin behandelt.

Die neuen Verbindungen der Formel (I) worin $R^1$ ein Alkyl mit 2 oder 3 C-Atomen darstellt können ferner erhalten werden, wenn man (d) eine Verbindung der Formel (VII)

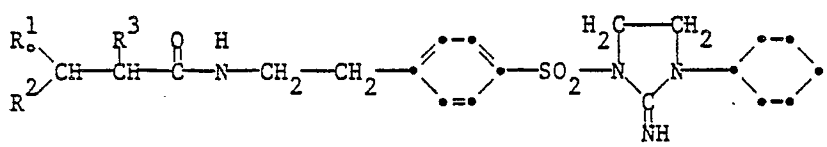

(VII)

in welchem $R^{1°}$ für einen 1-Alkenylrest mit 2-3 C-Atomen steht, oder ein Derivat davon, isomerisiert, und, wenn erwünscht, die zusätzlichen Verfahrensschritte durchführt.

Ein Derivat einer Verbindung der Formel (VII) ist in erster Linie ein Säureadditionssalz, wie ein Salz mit einer Mineral-, wie Halogenwasserstoffsäure.

Die Isomerisierungsreaktion erfolgt üblicherweise spontan, d. h. in situ im Zusammenhang mit der Herstellung der Ausgangsstoffe der Formel (VII), wenn notwendig oder erwünscht, unter Erwärmen und in Gegenwart eines geeigneten Lösungsmittels, in einem geschlossenen Gefäss und/oder in einer Inertgas-, z. B. Stickstoffatmosphäre.

Die Ausgangsstoffe können in an sich bekannter Weise erhalten werden, z. B. durch Behandeln einer Verbindung der Formel (II) mit einer Säure der Formel

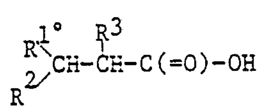

(VIII)

oder einem geeigneten Derivat, z. B. einem Anhydrid, wie Halogenid, z. B. Chlorid, davon, wenn notwendig, in Gegenwart eines Kondensationsmittels oder eines säurebindenden Mittels. Wie oben erwähnt, kann das Ausgangsmaterial der Formel (VII) spontan zu einer Verbindung der Formel (I) isomerisieren.

Verfahrensgemäss erhältliche Salze können in an sich bekannter Weise, z. B. durch Behandeln mit einer geeigneten Base, in die freien Verbindungen, oder z. B. durch Umsalzen mit einem geeigneten Salz, in ein anderes Salz übergeführt werden.

Erfindungsgemäss erhältliche freie Verbindungen der Formel (I) können gewünschtenfalls in ihre Säureadditionssalze, z. B. durch Umsetzen mit einer Säure in einem geeigneten Lösungsmittel übergeführt werden.

Erfindungsgemäss erhältliche Isomerengemische können in an sich bekannter Weise, z. B. mittels physikalisch-chemischer Trennverfahren in die einzelnen Isomeren aufgetrennt werden, wobei man vorzugsweise das aktivere Isomere isoliert.

Die Erfindung betrifft auch diejenigen Ausführungsformen des Verfahrens, nach denen man von einer, auf irgendeiner Stufe des Verfahrens als Zwischenprodukt erhältlichen Verbindung ausgeht und die fehlenden Schritte durchführt oder einen Ausgangsstoff in Form eines Derivates, z. B. Salzes, und/oder eines isomeren oder Isomerengemisches verwendet oder, wie oben gezeigt, unter den Reaktionsbedingungen bildet.

Bei dem Verfahren der vorliegenden Erfindung werden vorzugsweise solche Ausgangsstoffe verwendet, welche zu den eingangs als besonders wertvoll geschilderten Verbindungen führen.

Die Verbindungen der vorliegenden Erfindung weisen pharmakologische, in erster Linie ausgeprägte blutzuckersenkende Eigenschaften auf, die sich sowohl an normalen, als auch an diabetischen Versuchstieren nachweisen lassen.

Die Bestimmung der blutzuckersenkenden Wirkung an normalen Versuchstieren wird an normalen weiblichen Ratten (Tif : RAIF (SPF)) mit einem Körpergewicht von 170 bis 200 g durchgeführt, denen 6,5 Stunden vor Versuchsbeginn das Futter entzogen wird. Nach einer ersten Blutentnahme (Ausgangswert) wird das Versuchspräparat oral (mittels Schlundsonde) als Suspension in 0,5 %iger wässriger Lösung von Methylcellulose und in einem Volumen von 5 ml/kg Körpergewicht verabreicht. Weitere Blutentnahmen erfolgen nach 2, 4, 5 und 7,5 Stunden, wobei pro Dosis mindestens 5 Versuchstiere verwendet werden. Die Blutentnahme wird retroorbital (Riley, Proc. Soc. Exptl. Biol. Med., Bd. 104, S. 751 (1960)) bei leichter Narkose (Sauerstoff/Kohlendioxid 1 : 1, 45 Sekunden ; Green, Animal Handbooks 8, London Laboratory Animals Ltd, S. 154 (1979)) durchgeführt. Die Blutzuckerbestimmung erfolgt nach der enzymatischen GOD-Methode (Werner et al., Z. anal. Chem., Bd. 252, S. 224 (1970)) auf dem Autoanalyzer.

In dieser Versuchsanordnung zeigen Verbindungen der vorliegenden Erfindung folgende blutzuckersenkenden Wirkungen :

| Verbindung (Beispiel) | Maximale Blutzuckersenkung (in % des Ausgangswertes) bei den angegebenen Dosen (in mg/kg p.o.) | | | |
|---|---|---|---|---|
| | 30 | 10 | 3 | 1 |
| 1 | 80 | 76 | 41 | 17 |
| 10 | - | 43 | 23 | 11 |
| 11 | 80 | 59 | 31 | 14 |
| 12 | 64 | 48 | 12 | - |
| 13 | 79 | 51 | 11 | - |

Die Bestimmung der blutzuckersenkenden Wirkung an diabetischen Versuchstieren wird an Streptozotocin-diabetischen Ratten durchgeführt, wobei in männlichen Ratten (Tif : RAIF (SPF)) mit einem Gewicht von 140 bis 200 g, denen 16 bis 24 Stunden vor Versuchsbeginn das Futter entzogen wird, durch eine einmalige Injektion von Streptozotocin (55 mg/kg) in die Schwanzvene eine Streptozotocindiabetes erzeugt wird. Versuchstiere mit Blutzuckerwerten zwischen 300 und 700 mg/Prozenten (etwa 17 bis 39 mMol/lt) werden frühestens 3 bis 4 Wochen nach dieser Behandlung für die Versuche verwendet.

Die Blutzuckerbestimmungen werden 3 bis 6 Stunden nach der oralen Verabreichung des Versuchspräparates (siehe oben für die Normalratte) an gefütterten Tieren durchgeführt. Da der Blutzucker bei diabetischen Ratten, im Gegensatz zu normalen Ratten, während der Versuchsdauer in der Regel 5 bis 15 % abfällt, wird die spontane Blutzuckersenkung (in % des Ausgangswertes) einer gleichzeitig untersuchten Kontrollgruppe von der Blutzuckersenkung der Testgruppe in Abzug gebracht. Dabei werden pro Gruppe mindestens 5 Tiere verwendet.

In dieser Versuchsanordung zeigt z. B. die Verbindung des Beispiels 1 bei einer Dosis von 100 mg/kg eine maximale Blutzuckersenkung (in % des Ausgangswertes) von 29 % und bei einer Dosis von 30 mg/kg eine solche von 10 %.

Zudem weisen die Verbindungen der vorliegenden Erfindung eine, im Vergleich mit den starken

antidiabetischen Wirkungen, geringe Toxizität auf. So beträgt z. B. die $LD_{50}$-Dosis für die Verbindung des Beispiels 1 600 mg/kg p. o. (Ratte).

Die Verbindungen der vorliegenden Erfindung können deshalb zur Senkung des Blutzuckerspiegels bei Diabetes verwendet werden, wobei sie sich, wegen des schnellen Wirkungseintritts und der bemerkenswert kurzen Wirkungsdauer (das Wirkungsmaximum wird z. B. mit einer Dosis von 2 mg/kg der Verbindung des Beispiel 1 nach 1 Stunde erreicht und die Wirkung kann nach 5 Stunden nicht mehr festgestellt werden), in erster Linie zur periprandialen Behandlung von Altersdiabetes eignen.

Die Erfindung betrifft ebenfalls die Verwendung der Verbindungen der Formel (I) oder von pharmazeutisch verwendbaren Salzen von solchen Verbindungen mit salzbildenden Eigenschaften, insbesondere als pharmakologisch wirksame, besonders als blutzuckersenkende Verbindungen. Dabei kann man sie, vorzugsweise in Form von pharmazeutischen Präparaten, in einem Verfahren zur prophylaktischen und/oder therapeutischen Behandlung des tierischen oder menschlichen Körpers, insbesondere zur Behandlung von Diabetes verwenden. Die Dosierung des Wirkstoffs, der allein oder zusammen mit dem üblichen Träger- und Hilfsmaterial verabreicht wird, hängt von der zu behandelnden Spezies, deren Alter und individuellen Zustand, sowie der Verabreichungsweise ab. Die täglichen Dosen liegen für Mammalien mit einem Körpergewicht von etwa 70 kg, je nach Art des Diabetes, individuellem Zustand und Alter, vorzugsweise zwischen etwa 50 bis 500 mg.

Die Erfindung betrifft im weiteren pharmazeutische Präparate, die Verbindungen der Formel (I) oder pharmazeutisch verwendbare Salze davon als Wirkstoffe enthalten, sowie Verfahren zu ihrer Herstellung.

Bei den erfindungsgemässen pharmazeutischen Präparaten handelt es sich um solche zur enteralen, wie peroralen oder rektalen, weiter zur sublingualen, sowie zur parenteralen Verabreichung an Warmblüter. Entsprechende Dosiseinheitsformen, insbesondere zur peroralen und/oder sublingualen Verabreichung, z. B. Dragées, Tabletten oder Kapseln, enthalten vorzugsweise von etwa 50 bis etwa 250 mg, insbesondere von etwa 50 bis etwa 150 mg einer Verbindung der Formel (I) oder eines pharmazeutisch annehmbaren Salzes davon zusammen mit pharmazeutisch verwendbaren Trägerstoffen.

Geeignete Trägerstoffe sind insbesondere Füllstoffe, wie Zucker, z. B. Lactose, Saccharose, Mannit oder Sorbit. Cellulosepräparate und/oder Calciumphosphate, z. B. Tricalciumphosphat oder Calciumhydrogenphosphat, ferner Bindemittel, wie Stärkekleister unter Verwendung z. B. von Mais-, Weizen-, Reis- oder Kartoffelstärke, Gelatine, Tragacanth oder Methylcellulose, und/oder, wenn erwünscht, Sprengmittel, wie die obengenannten Stärken, ferner Carboxymethylstärke, quervernetztes Polyvinylpyrrolidon, Agar, Alginsäure oder ein Salz davon, wie Natriumalginat. Hilfsmittel sind in erster Linie Fliessregulier- und Schmiermittel, z. B. Kieselsäure, Talk, Stearinsäure oder Salze davon, wie Magnesium- oder Calciumstearat, und/oder Polyäthylenglykol.

Dragée-Kerne können mit geeigneten, gegebenenfalls Magensaft-resistenten Ueberzügen versehen werden, wobei man u. a. konzentrierte Zuckerlösungen, welche gegebenenfalls arabischen Gummi, Talk, Polyvinylpyrrolidon, Polyäthylenglykol und/oder Titandioxid enthalten, oder Lacklösungen in geeigneten organischen Lösungsmitteln oder Lösungsmittelgemischen, oder, zur Herstellung von Magensaft-resistenten Ueberzügen, Lösungen von geeigneten Cellulosepräparaten, wie Acetylcellulosephthalat oder Hydroxypropylmethylcellulosephthalat, verwendet.

Den Tabletten oder Dragée-Ueberzügen können Farbstoffe oder Pigmente, z. B. zur Identifizierung oder zur Kennzeichnung verschiedener Wirkstoffdosen, beigefügt werden.

Weitere oral anwendbare pharmazeutische Präparate sind Steckkapseln aus Gelatine, sowie weiche, geschlossene Kapseln aus Gelatine und einem Weichmacher, wie Glycerin oder Sorbit. Die Steckkapseln können den Wirkstoff in Form eines Granulats, z. B. im Gemisch mit Füllstoffen, wie Lactose, Bindemitteln, wie Stärken und/oder Gleitmitteln, wie Talk oder Magnesiumstearat, und gegebenenfalls von Stabilisatoren, enthalten. In weichen Kapseln ist der Wirkstoff vorzugsweise in geeigneten Flüssigkeiten, wie Oelen, Paraffinöl oder flüssigen Polyäthylenglykolen, gelöst oder suspendiert, wobei ebenfalls Stabilisatoren zugefügt sein können.

Als rektal anwendbare pharmazeutische Präparate kommen z. B. Suppositorien in Betracht, welche aus einer Kombination des Wirkstoffs mit einer Suppositoriengrundmasse bestehen. Als Suppositoriengrundmasse eignen sich z. B. natürliche oder synthetische Triglyceride, Paraffinkohlenwasserstoffe, Polyäthylenglykole oder höhere Alkanole. Ferner können auch Gelatine-Rektalkapseln verwendet werden, die eine Kombination des Wirkstoffes mit einer Grundmasse enthalten ; als Grundmassenstoffe kommen z. B. flüssige Triglyceride, Polyäthylenglykole oder Paraffinkohlenwasserstoffe in Frage.

Zur parenteralen Verabreichung eignen sich in erster Linie wässerige Lösungen eines Wirkstoffes in wasserlöslicher Form, z. B. eines wasserlöslichen Salzes, ferner Suspensionen des Wirkstoffes, wie entsprechende ölige Injektionssuspensionen, wobei man geeignete lipophile Lösungsmittel oder Vehikel, wie fette Oele, z. B. Sesamöl, oder synthetische Fettsäureester, z. B. Aethyloleat, oder Triglyceride verwendet, oder wässrige Injektionssuspensionen, welche viskositätserhöhende Stoffe, z. B. Natriumcarboxymethylcellulose, Sorbit und/oder Dextran und gegebenenfalls Stabilisatoren enthalten.

Die pharmazeutischen Präparate der vorliegenden Erfindung können in an sich bekannter Weise, z. B. mittels konventioneller Misch-, Granulier-, Dragier-, Lösungs- oder Lyophilisierungsverfahren hergestellt werden. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig, nach Zugabe von geeigneten Hilfsstoffen,

zu Tabletten oder Dragée-Kernen verarbeitet.

Die nachfolgenden Beispiele illustrieren die oben beschriebene Erfindung. Temperaturen werden in Celsiusgraden angegeben.

Beispiel 1

Zu einer Lösung von 30 g Crotonsäure in 1 500 ml Methylenchlorid werden bei — 10° nacheinander 51 ml Triäthylamin und 34 ml Chlorameisensäureäthylester zugetropft. Nach 20 Minuten wird bei dieser Temperatur eine Suspension von 153 g 1-[4-(2-Aminoäthyl)-phenylsulfonyl]-2-imino-3-cyclohexyl-imidazolidin-di-hydrochlorid in 1 500 ml Methylenchlorid zugegeben und bei 0° eine Lösung von 107 ml Triäthylamin in 500 ml Methylenchlorid zugetropft. Es wird noch 3 Stunden bei 0° und 12 Stunden bei Raumtemperatur weiter gerührt, wobei die Lösung klar wird. Sie wird nacheinander mit je 500 ml Wasser, gesättigter wässriger Natriumhydrogencarbonat-Lösung und Wasser gewaschen. Die organische Phase wird über Natriumsulfat getrocknet, filtriert und unter vermindertem Druck zur Trockne eingedampft. Das so erhältliche 1-{4-[2-(Crotonylamino)-äthyl]-phenylsulfonyl}-3-cyclohexyl-2-imino-imidazolidin (trans) wird aus Essigsäureäthylester umkristallisiert und schmilzt bei 157 bis 158°.

Beispiel 2

Zu einer Lösung von 3.5 g 1-[4-(2-Amino-äthyl)-phenylsulfonyl]-2-imino-3-cyclohexyl-imidazolidin in 100 ml absolutem Dioxan wird bei 0° eine Lösung 1,5 g Crotonsäureanhydrid in 5 ml absolutem Dioxan zugetropft. Nach 12-stündigem Rühren bei Raumtemperatur wird das Dioxan abdestilliert, der Rückstand in 300 ml Methylenchlorid gelöst, und die Lösung nacheinander mit je 50 ml Wasser, 0,5-n. wässrigem Natriumhydroxid und Wasser gewaschen. Die organische Phase wird über Natriumsulfat getrocknet, filtriert und unter vermindertem Druck zur Trockne eingedampft. Man erhält so das 1-{4-[2-(Crotonylamino)-äthyl]-phenylsulfonyl}-3-cyclohexyl-2-imino-imidazolidin (trans), das, aus Essigsäureäthylester umkristallisiert bei 157 bis 158° schmilzt.

Beispiel 3

Eine Lösung von 2,2 g Vinylessigsäure wird bei — 10° nacheinander mit 2,7 g Triäthylamin 2,7 g Chlorameisensäureäthylester und einer Lösung von 7,4 g 1-[4-(2-Amino-äthyl)-phenylsulfonyl]-2-imino-3-cyclohexyl-imidazolidin in 100 ml Methylenchlorid versetzt. Nach 3-stündigem Rühren bei 0° lässt man das Reaktionsgemisch über Nacht stehen und wäscht dann nacheinander mit Wasser, gesättigter wässriger Natriumhydrogencarbonatlösung und Wasser. Nach Umkristallisieren aus Essigsäureäthylester erhält man das 1-{4-[2-Crotonyl-amino)-äthyl]-phenylsulfonyl}-3-cyclohexyl-2-imino-imidazolidin (trans), Smp. 157 bis 158°.

Beispiel 4

Man fügt 20,4 g 1-Cyclohexyl-2-imino-imidazolidin-hydrochlorid zu 8,5 g Natriumhydroxid in 85 ml Wasser. Die erhaltene Lösung wird mit 28,8 g in 100 ml Aceton gelöstem 4-(2-Crotonylamino-äthyl)-benzolsulfochlorid versetzt, wobei sich das Reaktionsgemisch erwärmt. Es wird während einer halben Stunde auf 90° erhitzt und dann unter vermindertem Druck eingedampft. Man kristallisiert den Rückstand aus Essigsäureäthylester um und erhält 1-{4-[2-(Crotonyl-amino)-äthyl]-phenylsulfonyl}-3-cyclohexyl-2-imino-imidazolidin (trans), das bei 157 bis 158° schmilzt.

Das Ausgangsmaterial kann wie folgt hergestellt werden :

18,9 g N-(2-Phenyl-äthyl)-crotonsäureamid werden portionsweise unter Rühren zu 35,0 g Chlorsulfonsäure gegeben. Anschliessend wird das Gemisch während 3 Stunden bei 60° gerührt, worauf es auf Eis gegossen wird. Die Kristalle werden abgenutscht, mit Wasser gewaschen und unter vermindertem Druck getrocknet. Das erhaltene 4-(2-Crotonylamino-äthyl)-benzolsulfonylchlorid wird als Rohprodukt weiterverarbeitet.

Beispiel 5

Ein Gemisch von 28 ml 4-n. wässriger Natriumhydroxidlösung und 4,3 g Aethylenimin werden auf — 10° gekühlt. Hierauf wird eine Suspension von 28,8 g 4-(2-Crotonylamino-äthyl)-benzolsulfochlorid in 100 ml Aceton unter Rühren und Kühlen zugegeben, sodass die Temperatur nicht über 0° steigt. Nach beendetem Zutropfen wird während 30 Minuten bei 0° weitergerührt. Dann wird das Kühlbad entfernt und die Lösung des so erhältlichen 1-[4-(2-Crotonylamino-äthyl)-phenylsulfonyl]-aziridins mit 100 ml Cyclohexylamin versetzt. Die Temperatur steigt auf 40-50°. Das Reaktionsgemisch wird während 1 Stunde weitergerührt und anschliessend im Rotationsverdampfer das überschüssige Amin abdestilliert. Der erhaltene Kristallbrei, der neben Kochsalz das gewünschte 1-[4-(2-Crotonylamino-äthyl)-phenylsulfonyl]-2-cyclohexyl-äthylendiamin enthält, wird in 56 ml 2-n. wässrigen Natriumhydroxid gelöst und unter Rühren portionsweise mit 10,6 g Bromcyan versetzt, wobei man die Temperatur nicht über 40° steigen

lässt. Nach einer Stunde wird mit Methylenchlorid extrahiert, die organische Lösung mit 20 ml 2-n. wässrigem Natriumhydroxid und zweimal mit 100 ml Wasser gewaschen. Nach dem Trocknen und Eindampfen der Methylenchloridlösung unter vermindertem Druck wird der Rückstand aus Essigsäureäthylester umkristallisiert und ergibt 1-{4-[2-(Crotonylamino)-äthyl]-phenylsulfonyl}-3-cyclohexyl-2-imino-imidazolidin (trans) vom Smp. 157 bis 158°.

Beispiel 6

Zu einer Lösung von 10,6 g Bromcyan in 100 ml absolutem Diäthyläther wird bei —5° eine Lösung von 19,8 g Cyclohexylamin in 400 ml absolutem Diäthyläther eingetropft. Nach beendigter Zugabe rührt man während 30 Minuten nach, filtriert vom ausgefallenen Cyclohexylamin-hydrobromid ab und trägt unter Feuchtigkeitsausschluss bei —5° 5,3 g einer 50 %igen Suspension von Natriumhydrid in Mineralöl portionenweise in das Filtrat ein. Man rührt nach beendigter Zugabe noch während 30 Minuten bei —5° und lässt die Temperatur dann auf 20° steigen. In die so erhaltene Suspension von Cyclohexylcyanamidnatrium tropft man innerhalb von 15 Minuten eine Lösung von 33,1 g N-(2-Chlor-äthyl)-4-(2-crotonylamino-äthyl)-benzolsulfonamid in 100 ml Dioxan ein, rührt die erhaltene Suspension während 15 Stunden bei Raumtemperatur und kocht dann während 5 Stunden am Rückfluss. Nach dem Abkühlen auf Zimmertemperatur tropft man 100 ml Wasser zu und dampft unter vermindertem Druck zur Trockne ein. Man verteilt den Rückstand zwischen Wasser und Chloroform, filtriert vom unlöslichen Anteil ab und trennt die beiden Phasen. Die Chloroform-Phase wird mit 2-n. Salzsäure extrahiert, der wässrig-saure Extrakt wird unter Kühlung mit konzentriertem wässrigem Natriumhydroxid alkalisch gestellt und mit Methylenchlorid extrahiert. Der Methylenchlorid-Extrakt wird mit Natriumsulfat getrocknet und das Methylenchlorid abdestilliert. Der Rückstand wird aus Essigsäureäthylester umkristallisiert und ergibt 1-{4-[2-(Crotonylamino)-äthyl]-phenylsulfonyl}-3-cyclohexyl-2-imino-imidazolidin (trans), das bei 157 bis 158° schmilzt.

Beispiel 7

Zu einer Lösung von 8,0 g Natriumhydroxid und 4,3 g Aethylenimin in 60 ml Wasser wird eine Lösung von 28,8 g 4-(2-Crotonylamino-äthyl)-benzolsulfochlorid in 100 ml Aceton zugetropft, wobei die Temperatur der Mischung durch Kühlen zwischen 0 und + 10° gehalten wird. Nach beendigter Zugabe wird während einer Stunde bei Raumtemperatur nachgerührt und dann das Aceton unter vermindertem Druck abgedampft. Aus der zurückbleibenden wässrigen Lösung scheidet sich das rohe N-[4-(2-Crotonylamino-äthyl)-phenylsulfonyl]-äthylenimin als Oel ab. Es wird mit Methylenchlorid extrahiert, der Extrakt wird mit Natriumsulfat getrocknet und das Methylenchlorid unter vermindertem Druck abgedampft. Das N-[4-(2-Crotonylamino-äthyl)-phenylsulfonyl]-äthylenimin bleibt als kristalliner Rückstand zurück.

Eine Lösung von 10,6 g Bromcyan in 100 ml absolutem Diäthyläther wird unter Rühren bei —5° in eine Lösung von 19,8 g Cyclohexylamin in 400 ml absolutem Diäthyläther eingetropft. Nach 30 Minuten wird das ausgefallene Cyclohexlamin-hydrobromid abfiltriert und das Filtrat portionsweise bei —5° mit 5,3 g einer Natriumhydrid-Mineralöl-Suspension (50 %ig) versetzt. Man rührt noch während 30 Minuten bei —5° und lässt dann die Temperatur auf etwa 20° ansteigen. In die so erhaltene, weisse Suspension von Cyclohexylcyanamidnatrium trägt man unter Rühren eine Lösung des nach dem vorstehenden Verfahren hergestellten N-[4-(2-Crotonylamino-äthyl)-phenylsulfonyl]-äthylenimins in 150 ml Dioxan ein. Die erhaltene Suspension wird während 15 Stunden bei Raumtemperatur gerührt und dann während 5 Stunden am Rückfluss gekocht. Nach dem Abkühlen auf Raumtemperatur tropft man 50 ml Wasser zu und dampft unter vermindertem Druck zur Trockne ein. Der Rückstand wird zwischen Chloroform und konzentriertem wässrigem Natriumhydroxid verteilt. Die Chloroform-Phase wird von unlöslichen Harzen abdekantiert, mit 2-n. Salzsäure extrahiert, und der Extrakt mit konzentriertem wässrigem Natriumhydroxid alkalisch gestellt und mit Methylenchlorid extrahiert. Die organische Phase wird über Natriumsulfat getrocknet, filtriert und unter vermindertem Druck zur Trockne eingedampft. Das verbleibende 1-{4-[2-(Crotonylamino)-äthyl]-phenylsulfonyl}-3-cyclohexyl-2-imino-imidazolidin (trans) wird aus Essigsäureäthylester umkristallisiert und schmilzt bei 157-158°.

Beispiel 8

Zu einem Gemisch von 4 g Natriumhydroxid 15 ml Wasser und 300 ml Dimethylsulfoxid gibt man 26,8 g 4-(2-Crotonylamino-äthyl)-benzolsulfonamid und 18,7 g N-(2-Chlor-äthyl)-N-cyclohexylcyanamid. Die erhaltene Lösung wird während 1 Stunde bei einer Badtemperatur von 110° erwärmt. Das Dimethylsulfoxid wird unter vermindertem Druck abdestilliert und der Rückstand ein braunes Oel, in Methylenchlorid aufgenommen. Man wäscht dreimal mit Wasser, trocknet und filtriert die organische Phase, die nach dem Eindampfen unter vermindertem Druck einen braunen kristallinen Rückstand ergibt. Nach Kristallisation aus Essigsäureäthylester erhält man das 1-{4-[2-(Crotonylamino)-äthyl]-phenylsulfonyl}-3-cyclohexyl-2-imino-imidazolidin (trans) vom Smp. 157 bis 158°.

Beispiel 9

Eine Lösung von 9,0 g Dinatrium-cyanamid in 50 ml Wasser wirdzunächst mit 15 ml Aceton und dann

portionsweise innerhalb von 15 Minuten mit 29,8 g 4-(2-Crotonylamino-äthyl)-benzol-sulfochlorid versetzt. Darauf fügt man 19,8 g 2-Chlor-äthyl-cyclohexylamin-hydrochlorid zu und dampft zur Trockne ein. Der breiartige Rückstand wird mit einem Gemisch von 150 ml Aethanol und 150 ml Isopropanol extrahiert. Der Extrakt wird unter vermindertem Druck zur Trockne eingedampft, der Rückstand in 200 ml Aceton aufgenommen, die Lösung filtriert und das Filtrat wiederum eingedampft. Das zurückbleibende, klare, gelbe Oel wird während 10 Stunden auf 145° erhitzt. Der nach dem Abkühlen zurückbleibende glasartige Rückstand wird in Wasser aufgenommen, mit 2-n. Salzsäure angesäuert und die Lösung mit Chloroform gewaschen. Daraufhin wird die wässrige Phase unter Kühlen mit konzentriertem wässrigem Natriumhydroxid alkalisch gestellt, wobei sich ein Oel abscheidet, das in Methylenchlorid aufgenommen wird. Die erhaltene Methylenchloridlösung wird mit Wasser gewaschen über Natriumsulfat getrocknet und unter vermindertem Druck zur Trockne eingedampft. Beim nachfolgenden Chromatographieren des Rückstandes an Silicagel wird mit Chloroform, enthaltend 2,5 % Methanol 1-{4-[2-(Crotonylamino)-äthyl]-phenylsulfonyl}-3-cyclohexyl-2-imino-imidazolidin(trans) eluiert, das nach Abdampfen des Lösungsmittels kristallisiert und nach Umkristallisieren aus Essigsäureäthylester bei 157 bis 158° schmilzt.

Beispiel 10

2 g Isocrotonsäure werden in 100 ml Methylenchlorid gelöst. Bei —10° werden 3,4 ml Triäthylamin, 2,4 ml Chlorameisensäureäthylester und nach 20 Minuten eine Lösung von 10,2 g 1-[4-(2-Amino-äthyl)-phenylsulfonyl]-2-imino-3-cyclohexyl-imidazolidin in 100 ml Methylenchlorid zugetropft. Man belässt das Reaktionsgemisch während 3 Stunden bei 0° und während 12 Stunden bei Raumtemperatur, verdünnt dann mit Methylenchlorid und wäscht nacheinander mit Wasser, einer gesättigten wässrigen Natriumhydrogencarbonatlösung und Wasser. Die organische Phase wird über Natriumsulfat getrocknet, filtriert und unter vermindertem Druck eingedampft. Das verbleibende Oel wird mit Petroläther kristallisiert und das so erhältliche 3-Cyclohexyl-2-imino-1-{4-[2-isocrotonylamino)-äthyl]-phenylsulfonyl}-imidazolidin (cis) aus Essigsäureäthylester umkristallisiert, Smp. 93 bis 94°.

Beispiel 11

Zu einer Lösung von 2,4 g Tiglinsäure in 100 ml Methylenchlorid werden bei —10° nacheinander 3,4 ml Triäthylamin, 2,4 ml Chlorameisensäureäthylester, eine Suspension von 10,2 g 1-[4-(2-Aminoäthyl)-phenylsulfonyl]-2-imino-3-cyclohexyl-imidazolidindihydrochlorid in 100 ml Methylenchlorid und wieder 7 ml Triäthylamin in 100 ml Methylenchlorid zugetropft. Man lässt langsam auf Zimmertemperatur erwärmen und rührt während 12 Stunden weiter. Danach wird das Reaktionsgemisch mit Wasser, einer gesättigten wässrigen Natriumhydrogencarbonatlösung und Wasser gewaschen. Nach Trocknen der organischen Phase über Natriumsulfat wird unter vermindertem Druck eingedampft und das verbleibende Oel aus Essigsäureäthylester umkristallisiert. Man erhält so das 3-Cyclohexyl-2-imino-1-{4-[2-(2,3-dimethyl-acryl-amino)-äthyl]-phenylsulfonyl}-imidazolidin (trans) vom Schmelzpunkt 140-142°.

Beispiel 12

Zu einer Lösung von 2,8 g der trans 4-Methyl-2-pentensäure in 100 ml Methylenchlorid werden bei —10° 3,4 ml Triäthylamin und 2,4 ml Chlorameisensäureäthylester zugetropft, und nach 30 Minuten tropft man eine Lösung von 8,4 g 1-[4-(2-Amino-äthyl)-phenylsulfonyl]-2-imino-3-cyclohexyl-imidazolidin in 100 ml Methylenchlorid zu und rührt während 3 Stunden bei 0° und während 12 Stunden bei Raumtemperatur. Nach Filtrieren wird das Filtrat mit je 50 ml Wasser, einer gesättigten wässrigen Natriumhydrogencarbonatlösung und Wasser gewaschen, über Natriumsulfat getrocknet, erneut filtriert und unter vermindertem Druck eingedampft. Das so erhaltene 3-Cyclohexyl-2-imino-1-{4-[2-(4-methyl-2-pentenoyl-amino)-äthyl]-phenylsulfonyl}-2-imino-3-cyclohexyl-imidazolidin (trans) wird aus Essigsäureäthylester umkristallisiert, Smp. 160 bis 162°.

Beispiel 13

Zu einer Lösung von 2,7 g trans-2-Hexensäure in 100 ml Methylenchlorid werden bei —10° 3,4 g ml Triäthylamin und 2,4 ml Chlorameisensäureäthylester getropft. Nach 20 Minuten wird eine Suspension von 10,2 g 1-[4-(2-Amino-äthyl)-phenylsulfonyl]-2-imino-3-cyclohexyl-imidazolidin-dihydrochlorid in 100 ml Methylenchlorid und eine Lösung von 5 g Triäthylamin in 100 ml Methylenchlorid bei 0° zugetropft. Man lässt während 3 Stunden bei 0° und während 12 Stunden bei Raumtemperatur nachreagieren, wäscht das Reaktionsgemisch mit Wasser, trocknet über Magnesiumsulfat, filtriert und dampft unter vermindertem Druck ein. Der weisse, kristalline Rückstand wird aus Essigsäureäthylester umkristallisiert. Das so erhältliche 3-Cyclohexyl-2-imino-1-{4-[2-(2-hexenoylamino)-äthyl]-phenylsulfonyl}-imidazolidin (trans) schmilzt dann bei 153 bis 156°.

Beispiel 14

Tabletten, enthaltend 100 mg 1-{4-[2-(Crotonylamino)-äthyl]-phenylsulfonyl}-3-cyclohexyl-2-imino-

imidazolidin (trans), können wie folgt hergestellt werden :

Zusammensetzung (für 10 000 Tabletten).

| | |
|---|---|
| 1-{4-[2-(Crotonylamino)-äthyl]-phenylsulfonyl}-3-cyclohexyl-2-imino-imidazolidin (trans) | 1 000,0 g |
| Lactose | 500,0 g |
| Kartoffelstärke | 330,0 g |
| Gelatine | 8,0 g |
| Talk | 60,0 g |
| Magnesiumstearat | 10,0 g |
| Siliciumdioxid (kolloidal) | 20,0 g |
| Wasser | q. s. |

Das 1-{4-[2-(Crotonylamino)-äthyl]-phenylsulfonyl}-3-cyclohexyl-2-imino-imidazolidin (trans) wird mit der Lactose und 270,0 g der Kartoffelstärke vermischt, die Mischung mit einer wässrigen Lösung der Gelatine befeuchtet und durch ein Sieb granuliert. Nach dem Trocknen mischt man die restliche Kartoffelstärke, den Talk, das Magnesiumstearat und das kolloidales Siliciumdioxid zu und presst die Mischung zu Tabletten von 200 mg Gewicht, die gewünschtenfalls mit Teilkerben zur feineren Anpassung der Dosierung versehen sein können.

Beispiel 15

Dragées, enthaltend 100 mg 1-{4-[2-(Crotonylamino)-äthyl]-phenylsulfonyl}-3-cyclohexyl-2-imino-imidazolidin (trans), können wie folgt hergestellt werden :

Zusammensetzung (für 10 000 Dragées) :

| | |
|---|---|
| 1-{4-[2-(Crotonylamino)-äthyl]-phenylsulfonyl}-3-cyclohexyl-2-imino-imidazolidin (trans) | 1 000,0 g |
| Lactose | 500,0 g |
| Siliciumdioxid (kolloidal) | 290,0 g |
| Talk | 290,0 g |
| Kartoffelstärke | 40,0 g |
| Magnesiumstearat | 5,0 g |
| Saccharose (krist.) | 533,0 g |
| Schellack | 20,0 g |
| arabischer Gummi | 75,0 g |
| Farbstoff | 1,5 g |
| Wasser | q. s. |

Das 1-{4-[2-(Crotonylamino)-äthyl]-phenylsulfonyl}-3-cyclohexyl-2-imino-imidazolidin (trans) wird mit der Lactose und 270,0 g Siliciumdioxid, 40,0 g Talk, der Kartoffelstärke und dem Magnesiumstearat vermischt und zu Dragée-Kernen verpresst. Diese werden anschliessend mit einem konzentrierten wässrigen Sirup aus der Saccharose, dem Schellack, dem arabischem Gummi, dem restlichen Talk und Siliciumoxid und dem Farbstoff überzogen und getrocknet. Die erhaltenen Dragées weisen ein Gewicht von 240 mg auf.

**Patentansprüche** (für die Vertragsstaaten : BE, CH, LI, DE, FR, GB, IT, LU, NL, SE)

1. Verbindungen der Formel (I)

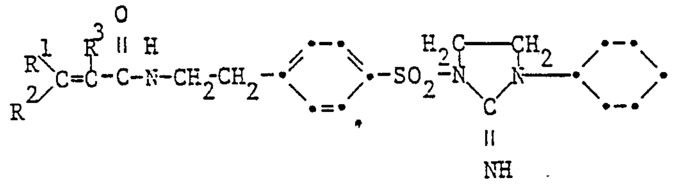

(I)

wobei $R^1$ für $C_{1-3}$-Alkyl und $R^2$ und $R^3$ unabhängig voneinander für Wasserstoff oder $C_{1-3}$-Alkyl stehen, und Salze von solchen Verbindungen.

2. Verbindungen nach Anspruch 1, wobei $R^1$ für Alkyl mit 1 bis 3 Kohlenstoffatomen sowie $R^2$ und $R^3$ für Wasserstoff oder Methyl stehen.

3. 1-{4-[2-(Crotonyl-amino)-äthyl]-phenylsulfonyl}-3-cyclohexyl-2-imino-imidazolidin.

4. 3-Cyclohexyl-2-imino-1-{4-[2-(isocrotonylamino)-äthyl]-phenylsulfonyl}-imidazolidin.

5. 3-Cyclohexyl-2-imino-1-{4-[2-(2,3-dimethyl-acryl-amino)-äthyl]-phenylsulfonyl}-imidazolidin (trans).

6. 3-Cyclohexyl-2-imino-1-{4-[2-(4-methyl-2-pentenoyl-amino)-äthyl]-phenylsulfonyl}-2-imino-3-cyclohexyl-imidazolidin (trans).

7. 3-Cyclohexyl-2-imino-1-{4-[2-(2-hexenoyl-amino)-äthyl]-phenyl-sulfonyl}-imidazolidin (trans).

8. Verbindungen der Ansprüche 1 bis 7 zur Anwendung in einem Verfahren zur therapeutischen Behandlung der menschlichen Körpers.

9. Die Verbindungen der Ansprüche 1 bis 7 als antidiabetisch wirksame Mittel.

10. Pharmazeutische Präparate enthaltend eine der Verbindungen der Ansprüche 1 bis 7.

11. Verwendung der Verbindungen der Ansprüche 1 bis 7 zur Herstellung von pharmazeutischen Präparaten.

12. Verwendung von Verbindungen gemäss Anspruch 1-7 zur Herstellung eines Arzneimittels zur Behandlung von Diabetes.

13. Verfahren zur Herstellung der Verbindungen gemäss Anspruch 1, dadurch gekennzeichnet, dass man

a) eine Verbindung der Formel (II)

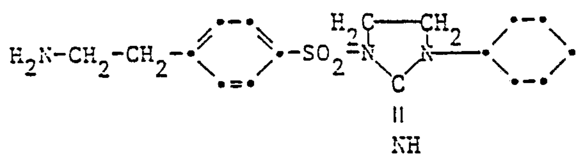

(II)

oder ein Derivat davon mit einer Säure der Formel (III)

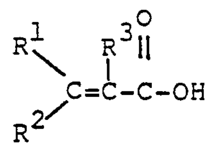

(III)

oder einem reaktionsfähigen Derivat davon umsetzt, oder

b) ein reaktionsfähiges Derivat einer Sulfonsäure der Formel (IV)

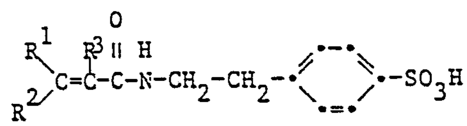

(IV)

mit einer Verbindung der Formel (V)

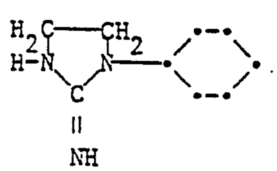

(V)

oder einem Derivat davon umsetzt, oder

c) eine Verbindung der Formel (VI)

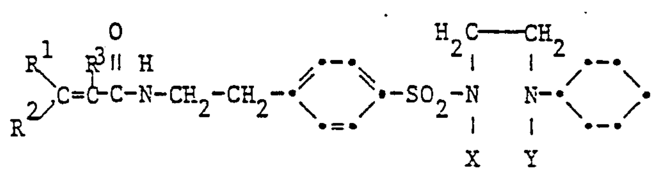

(VI)

worin einer der Reste X und Y Cyan und der andere Wasserstoff bedeuten, ringschliesst, und, wenn erwünscht, ein erfindungsgemäss erhältliches Salz in die freie Verbindung oder in ein anderes Salz, und/oder eine erfindungsgemäss erhältliche freie Verbindung in ein Salz umwandelt und/oder, wenn

erwünscht, ein Isomerengemisch in die einzelnen Isomeren auftrennt.

14. Verfahren zur Herstellung von Verbindungen gemäss Anspruch 1, worin $R_0^1$ Alkyl mit 2 oder 3 C-Atomen ist, dadurch gekennzeichnet, dass man eine Verbindung der Formel (VII)

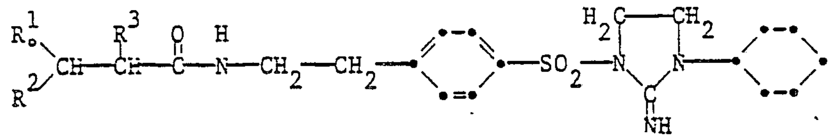

(VII)

in welcher $R_0^1$ für einen 1-Alkenylrest mit 2-3 C-Atomen steht, oder ein Derivat davon, isomerisiert, und, wenn erwünscht, ein erfindungsgemäss erhältliches Salz in die freie Verbindung oder in ein anderes Salz, und/oder eine erfindungsgemäss erhältliche freie Verbindung in ein Salz umwandelt und/oder, wenn erwünscht, ein Isomerengemisch in die einzelnen Isomeren auftrennt.

15. Verfahren nach Anspruch 13, dadurch gekennzeichnet, dass man das Ausgangsmaterial der Formel (VI) gemäss Verfahrensvariante (c) in situ bildet, indem man

ca) eine Verbindung der Formel (VIa)

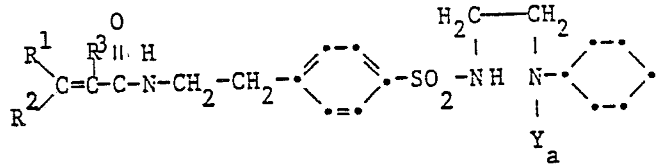

(VIa)

worin $Y_a$ für Wasserstoff oder einen unter den Reaktionsbedingungen abspaltbaren Rest steht, mit einem reaktionsfähigen Derivat der Cyansäure umsetzt, oder

cb) eine Verbindung der Formel (VIb)

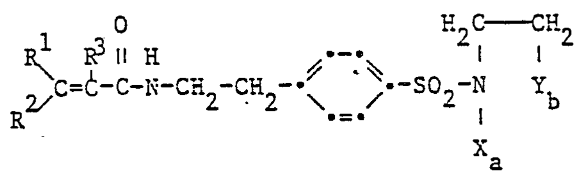

(VIb)

worin $X_a$ Wasserstoff und $Y_b$ eine reaktionsfähige veresterte Hydroxygruppe bedeuten, oder worin $X_a$ und $Y_b$ zusammen eine Bindung bilden, mit N-Cyclohexyl-cyanamid oder einem Derivat davon umsetzt, oder

cc) eine Verbindung der Formel (VIc)

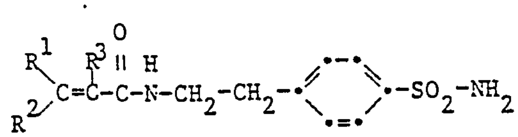

(VIc)

mit einem reaktionsfähigen veresterten N-Cyclohexyl-N-(2-hydroxy-äthyl)-cyanamid oder einem Derivat davon umsetzt, oder

cd) eine Additionssalz-Verbindung der Formel (VId)

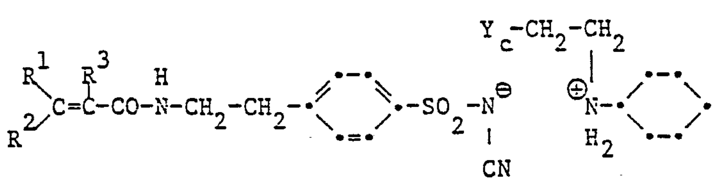

(VId)

worin $Y_c$ für eine reaktionsfähige veresterte Hydroxygruppe steht, cyclisiert.

**Patentansprüche** (für den Vertragsstaat AT)

1. Verfahren zur Herstellung einer Verbindung der Formel (I)

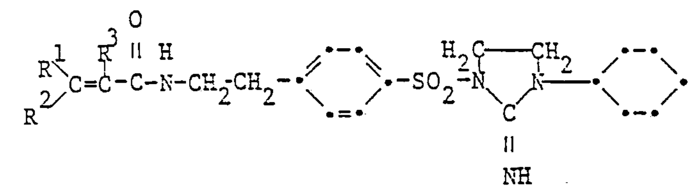 (I)

wobei $R^1$ für $C_{1-3}$-Alkyl und $R^2$ und $R^3$ unabhängig voneinander für Wasserstoff oder $C_{1-3}$-Alkyl stehen, und Salze von solchen Verbindungen, dadurch gekennzeichnet, dass man

a) eine Verbindung der Formel (II)

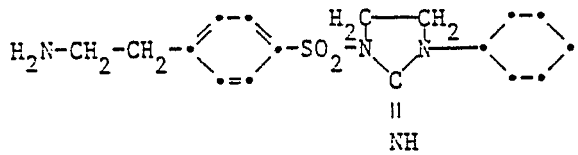 (II)

oder ein Derivat davon mit einer Säure der Formel (III)

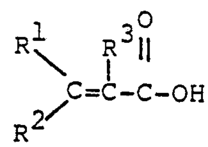 (III)

oder einem reaktionsfähigen Derivat davon umsetzt, oder

b) ein reaktionsfähiges Derivat einer Sulfonsäure der Formel (IV)

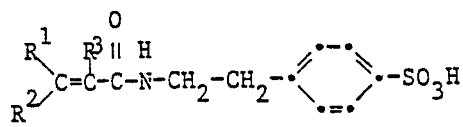 (IV)

mit einer Verbindung der Formel (V)

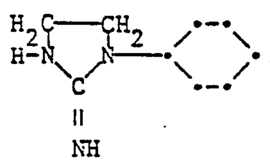 (V)

oder einem Derivat davon umsetzt, oder

c) eine Verbindung der Formel (VI)

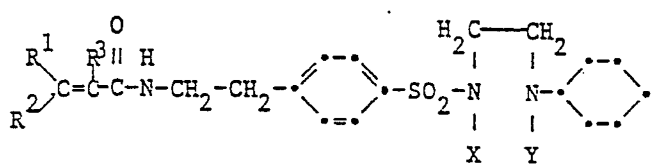 (VI)

worin einer der Reste X und Y Cyan und der andere Wasserstoff bedeuten, ringschliesst, und, wenn erwünscht, ein erfindungsgemäss erhältliches Salz in die freie Verbindung oder in ein anderes Salz, und/oder eine erfindungsgemäss erhältliche freie Verbindung in ein Salz umwandelt und/oder, wenn erwünscht, ein Isomerengemisch in die einzelnen Isomeren auftrennt.

2. Verfahren zur Herstellung von Verbindungen gemäss Anspruch 1, worin $R_0^1$ Alkyl mit 2 oder 3 C-Atomen ist, dadurch gekennzeichnet, dass man eine Verbindung der Formel (VII)

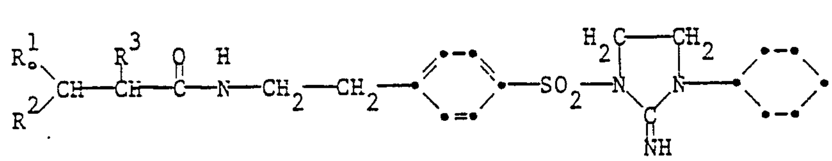 (VII)

in welcher $R_0^1$ für einen 1-Alkenylrest mit 2-3 C-Atomen steht, oder ein Derivat davon, isomerisiert, und, wenn erwünscht, ein erfindungsgemäss erhältliches Salz in die freie Verbindung oder in ein anderes Salz, und/oder eine erfindungsgemäss erhältliche freie Verbindung in ein Salz umwandelt und/oder, wenn erwünscht, ein Isomerengemisch in die einzelnen Isomeren auftrennt.

3. Verfahren nach Anspruch 1, dadurch gekennzeichnet, dass man das Ausgangsmaterial der Formel VI gemäss Verfahrensvariante (c) in situ bildet, indem man

ca) eine Verbindung der Formel (VIa)

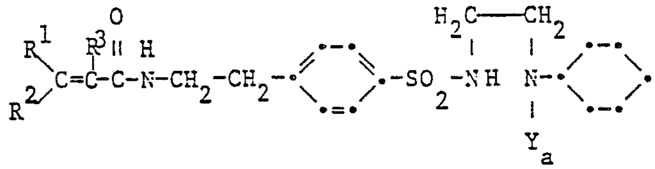

(VIa)

worin $Y_a$ für Wasserstoff oder einen unter den Reaktionsbedingungen abspaltbaren Rest steht, mit einem reaktionsfähigen Derivat der Cyansäure umsetzt, oder

cb) eine Verbindung der Formel (VIb)

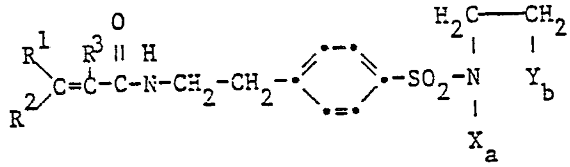

(VIb)

worin $X_a$ Wasserstoff und $Y_b$ eine reaktionsfähige veresterte Hydroxygruppe bedeuten, oder worin $X_a$ und $Y_b$ zusammen eine Bindung bilden, mit N-Cyclohexyl-cyanamid oder einem Derivat davon umsetzt, oder

cc) eine Verbindung der Formel (VIc)

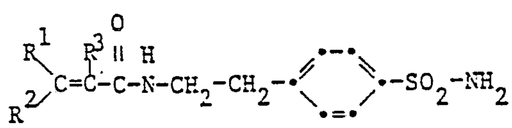

(VIc)

mit einem reaktionsfähigen veresterten N-Cyclohexyl-N-(2-hydroxy-äthyl)-cyanamid oder einem Derivat davon umsetzt, oder

cd) eine Additionssalz-Verbindung der Formel (VId)

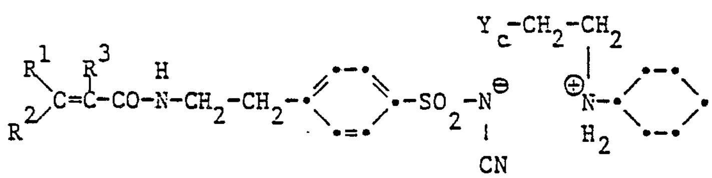

(VId)

worin $Y_c$ für eine reaktionsfähige veresterte Hydroxygruppe steht, cyclisiert.

4. Verfahren nach Patentanspruch 1, dadurch gekennzeichnet, dass man Verbindungen der Formel (I), worin $R^1$ für $C_{1-3}$-Alkyl, $R^2$ und $R^3$ für Wasserstoff oder Methyl stehen, oder Salze davon herstellt.

5. Verfahren nach Patentanspruch 1, dadurch gekennzeichnet, dass man, 1-{4-[2-(Crotonyl-amino)-äthyl]-phenylsulfonyl}-3-cyclohexyl-2-imino-imidazolidin herstellt.

6. Verfahren nach Patentanspruch 1, dadurch gekennzeichnet, dass man 3-Cyclohexyl-2-imino-1-{4-[2-(isocrotonylamino)-äthyl]-phenylsulfonyl}-imidazolidin herstellt.

7. Verfahren nach Patentanspruch 1, dadurch gekennzeichnet, dass man 3-Cyclohexyl-2-imino-1-{4-[2-(2,3-dimethyl-acryl-amino)-äthyl]-phenylsulfonyl}-imidazolidin (trans) herstellt.

8. Verfahren nach Patentanspruch 1, dadurch gekennzeichnet, dass man 3-Cyclohexyl-2-imino-1-{4-[2-(4-methyl-2-pentenoyl-amino)-äthyl]-phenylsulfonyl}-2-imino-3-cyclohexyl-imidazolidin (trans) herstellt.

9. Verfahren nach Patentanspruch 1, dadurch gekennzeichnet, dass man 3-Cyclohexyl-2-imino-1-{4-[2-(2-hexenoyl-amino)-äthyl]-phenylsulfonyl}-imidazolidin (trans) herstellt.

10. Verfahren zur Herstellung eines pharmazeutischen Präparates, gekennzeichnet durch die Verarbeitung eines nach den Ansprüchen 1-8 erfindungsgemäss gewonnenen Wirkstoffes, mit einem pharmazeutischen Trägermittel.

**Claims** (for the Contracting States : BE, CH, LI, DE, FR, GB, IT, LU, NL, SE)

1. Compounds of the formula (I)

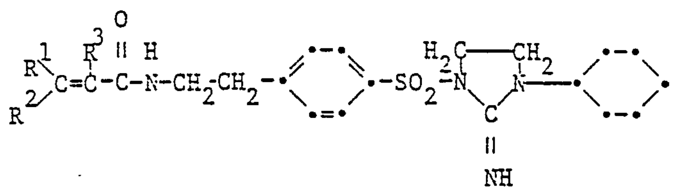

(I)

in which $R^1$ represents $C_{1\text{-}3}$-alkyl and each of $R^2$ and $R^3$, independently of the other, represents hydrogen or $C_{1\text{-}3}$-alkyl, and salts of such compounds.

2. Compounds according to claim 1, in which $R^1$ represents alkyl having from 1 to 3 carbon atoms, and $R^2$ and $R^3$ represent hydrogen or methyl.

3. 1-{4-[2-(crotonylamino)-ethyl]-phenylsulphonyl}-3-cyclohexyl-2-imino-imidazolidine.

4. 3-cyclohexyl-2-imino-1-{4-[2-(isocrotonylamino)-ethyl]-phenylsulphonyl}-imidazolidine.

5. 3-cyclohexyl-2-imino-1-{4-[2-(2,3-dimethylacrylamino)-ethyl]-phenylsulphonyl}-imidazolidine (trans).

6. 3-cyclohexyl-2-imino-1-{4-[2-(4-methyl-2-pentenoylamino)-ethyl]-phenylsulphonyl}-2-imino-3-cyclohexyl-imidazolidine (trans).

7. 3-cyclohexyl-2-imino-1-{4-[2-(2-hexenoylamino)-ethyl]-phenylsulphonyl}-imidazolidine (trans).

8. Compounds of claims 1 to 7 for use in a method for the therapeutic treatment of the human body.

9. The compounds of claims 1 to 7 as antidiabetically active agents.

10. Pharmaceutical preparations containing one of the compounds of claims 1 to 7.

11. The use of the compounds of claims 1 to 7 for the manufacture of pharmaceutical preparations.

12. The use of compounds of claims 1 to 7 for the manufacture of a medicament for the treatment of diabetes.

13. A process for the manufacture of the compounds according to claim 1, characterised in that

a) a compound of the formula (II)

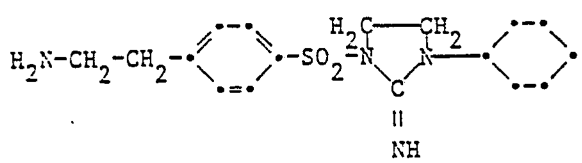

(II)

or a derivative thereof, is reacted with an acid of the formula (III)

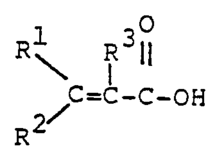

(III)

or with a reactive derivative thereof, or

b) a reactive derivative of a sulphonic acid of the formula (IV)

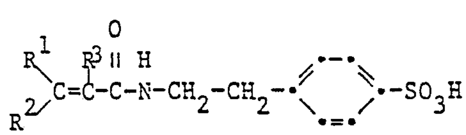

(IV)

is reacted with a compound of the formula (V)

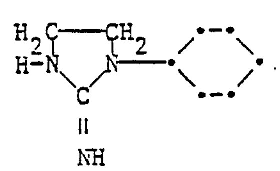

(V)

or with a derivative thereof, or

c) a compound of the formula (VI)

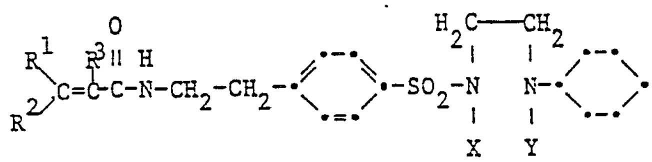

(VI)

in which one of the radicals X and Y represents cyano and the other represents hydrogen, is ring-closed, and, if desired, a salt obtainable according to the invention is converted into the free compound or into a different salt, and/or a free compound obtainable according to the invention is converted into a salt and/or, if desired, a mixture of isomers is separated into the individual isomers.

14. A process for the manufacture of compounds according to claim 1 in which $R_0^1$ is alkyl having 2 or 3 carbon atoms, characterised in that a compound of the formula (VII)

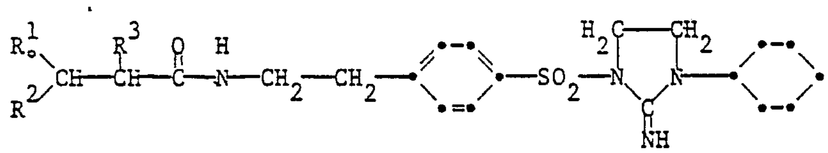

(VII)

in which $R_0^1$ represents a 1-alkenyl radical having 2 or 3 carbon atoms, or a derivative thereof, is isomerised, and, if desired, a salt obtainable according to the invention is converted into the free compound or into a different salt, and/or a free compound obtainable according to the invention is converted into a salt and/or, if desired, a mixture of isomers is separated into the individual isomers.

15. A process according to claim 13, characterised in that the starting material of the formula (VI) according to process variant (c) is formed in situ by

ca) reacting a compound of the formula (VIa)

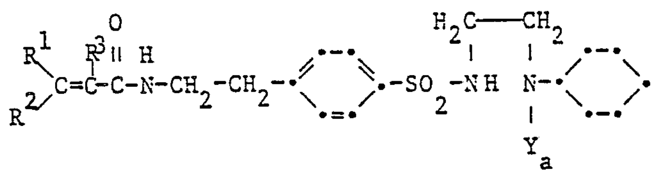

(VIa)

in which $Y_a$ represents hydrogen or a radical that can be removed under the reaction conditions, with a reactive derivative of cyanic acid, or

cb) reacting a compound of the formula (VIb)

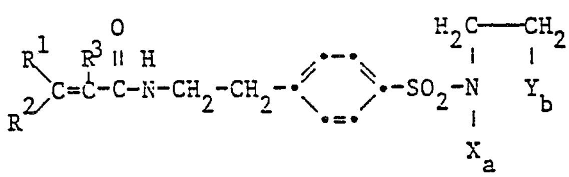

(VIb)

in which $X_a$ represents hydrogen and $Y_b$ represents a reactive esterified hydroxy group, or in which $X_a$ and $Y_b$ together form a bond, with N-cyclohexylcyanamide or a derivative thereof, or

cc) reacting a compound of the formula (VIc)

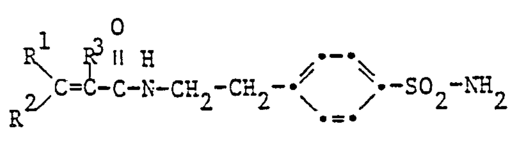

(VIc)

with a reactive esterified N-cyclohexyl-N-(2-hydroxy-ethyl)-cyanamide or a derivative thereof, or

cd) cyclising an addition salt compound of the formula (VId)

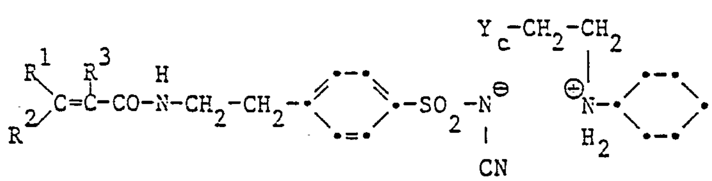

(VId)

in which $Y_c$ represents a reactive esterified hydroxy group.

**Claims** (for the Contracting State AT)

1. A process for the manufacture of a compound of the formula (I)

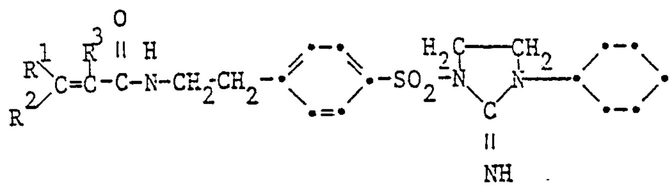

(I)

in which $R^1$ represents $C_{1-3}$-alkyl and each of $R^2$ and $R^3$, independently of the other, represents hydrogen or $C_{1-3}$-alkyl, and salts of such compounds, characterised in that

a) a compound of the formula (II)

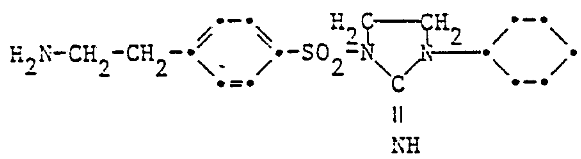

(II)

or a derivative thereof, is reacted with an acid of the formula (III)

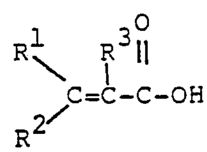

(III)

or with a reactive derivative thereof, or

b) a reactive derivative of a sulphonic acid of the formula (IV)

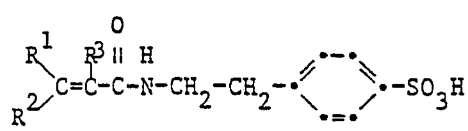

(IV)

is reacted with a compound of the formula (V)

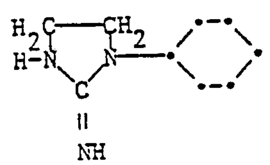

(V)

or with a derivative thereof, or

c) a compound of the formula (VI)

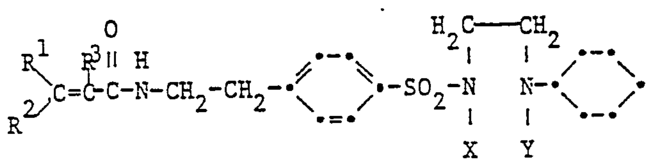

(VI)

in which one of the radicals X and Y represents cyano and the other represents hydrogen, is ring-closed, and, if desired, a salt obtainable according to the invention is converted into the free compound or into a different salt, and/or a free compound obtainable according to the invention is converted into a salt and/or, if desired, a mixture of isomers is separated into the individual isomers.

2. A process for the manufacture of compounds according to claim 1 in which $R_0^1$ is alkyl having 2 or 3 carbon atoms, characterised in that a compound of the formula (VII)

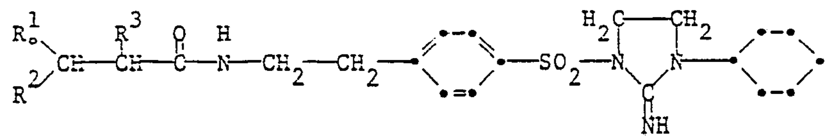

(VII)

in which $R_0^1$ represents a 1-alkenyl radical having 2 or 3 carbon atoms, or a derivative thereof, is isomerised, and, if desired, a salt obtainable according to the invention is converted into the free compound or into a different salt, and/or a free compound obtainable according to the invention is converted into a salt and/or, if desired, a mixture of isomers is separated into the individual isomers.

3. A process according to claim 1, characterised in that the starting material of the formula VI according to process variant (c) is formed in situ by

ca) reacting a compound of the formula (VIa)

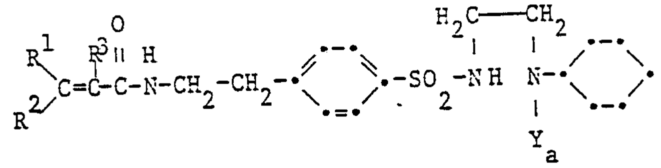

(VIa)

in which $Y_a$ represents hydrogen or a radical that can be removed under the reaction conditions, with a reactive derivative of cyanic acid, or

cb) reacting a compound of the formula (VIb)

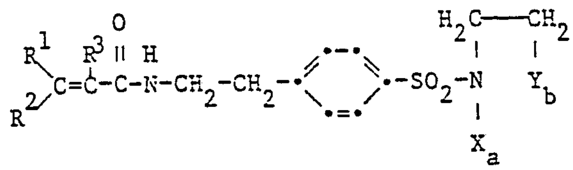

(VIb)

in which $X_a$ represents hydrogen and $Y_b$ represents a reactive esterified hydroxy group, or in which $X_a$ and $Y_b$ together form a bond, with N-cyclohexylcyanamide or a derivative thereof, or

cc) reacting a compound of the formula (VIc)

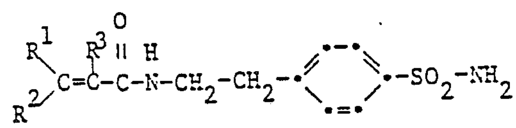

(VIc)

with a reactive esterified N-cyclohexyl-N-(2-hydroxy-ethyl)-cyanamide or a derivative thereof, or

cd) cyclising an addition salt compound of the formula (VId)

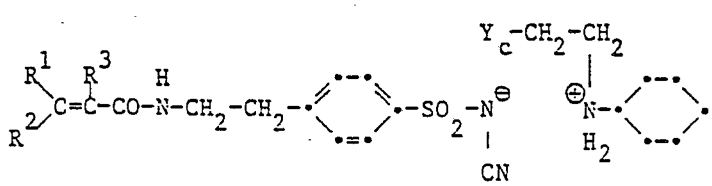

(VId)

in which $Y_c$ represents a reactive esterified hydroxy group.

4. A process according to patent claim 1, characterised in that compounds of the formula (I) in which $R^1$ represents $C_{1-3}$-alkyl, and $R^2$ and $R^3$ represent hydrogen or methyl, or salts thereof, are manufactured.

5. A process according to patent claim 1, characterised in that 1-{4-[2-(crotonylamino)-ethyl]-phenylsulphonyl}-3-cyclohexyl-2-imino-imidazolidine is manufactured.

6. A process according to patent claim 1, characterised in that 3-cyclohexyl-2-imino-1-{4-[2-(isocrotonylamino)-ethyl]-phenylsulphonyl}-imidazolidine is manufactured.

7. A process according to patent claim 1, characterised in that 3-cyclohexyl-2-imino-1-{4-[2-(2,3-dimethylacrylamino-ethyl]-phenylsulphonyl}-imidazolidine (trans) is manufactured.

8. A process according to patent claim 1, characterised in that 3-cyclohexyl-2-imino-1-{4-[2-(4-methyl-2-pentenoylamino)-ethyl]-phenylsulphonyl}-2-imino-3-cyclohexyl-imidazolidine (trans) is manufactured.

9. A process according to patent claim 1, characterised in that 3-cyclohexyl-2-imino-1-{4-[2-(2-hexenoylamino)-ethyl]-phenylsulphonyl}-imidazolidine (trans) is manufactured.

10. A process for the manufacture of a pharmaceutical preparation characterised by the processing of an active ingredient, obtained according to the invention in accordance with claims 1 to 8, with a pharmaceutical carrier.

**Revendications** (pour les Etats contractants : BE, CH, LI, DE, FR, GB, IT, LU, NL, SE)

1. Composés de formule I

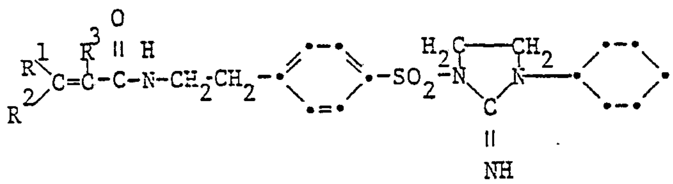

(I)

dans laquelle $R^1$ représente un groupe alkyle en C 1-C 3 et $R^2$ et $R^3$ représentent chacun, indépendamment l'un de l'autre, l'hydrogène ou un groupe alkyle en C 1-C 3, et les sels de ces composés.

2. Composés selon la revendication 1, dans lesquels $R^1$ représente un groupe alkyle en C 1-C 3 et $R^2$ et $R^3$ représentent l'hydrogène ou un groupe méthyle.

3. La 1-{4-[2-(crotonylamino)-éthyl]-phényl-sulfonyl}-3-cyclohexyl-2-imino-imidazolidine.

4. La 3-cyclohexyl-2-imino-1-{4-[2-(isocrotonylamino)-éthyl]-phénylsulfonyl}-imidazolidine.

5. La 3-cyclohexyl-2-imino-1-{4-[2-(2,3-diméthyl-acryl-amino)-éthyl]-phénylsulfonyl}-imidazolidine (trans).

6. La 3-cyclohexyl-2-imino-1-{4-[2-(4-méthyl-2-penténoyl-amino)-éthyl]-phénylsulfonyl}-2-imino-3-cyclohexyl-imidazolidine (trans).

7. La 3-cyclohexyl-2-imino-1-{4-[2-(2-hexénoylamino)-éthyl]-phénylsulfonyl}-imidazolidine (trans).

8. Composés des revendications 1 à 7, pour l'utilisation dans un procédé pour le traitement thérapeutique de l'organisme humain.

9. Les composés des revendications 1 à 7 en tant qu'agents à activité antidiabétique.

10. Compositions pharmaceutiques contenant l'un des composés des revendications 1 à 7.

11. Utilisation des composés des revendications 1 à 7 pour la préparation de compositions pharmaceutiques.

12. Utilisation des composés des revendications 1 à 7 pour la préparation d'un médicament prévu pour le traitement du diabète.

13. Procédé de préparation des composés selon la revendication 1, caractérisé en ce que

a) on fait réagir un composé de formule II

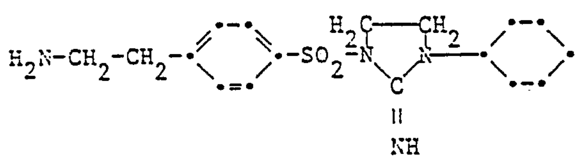

(II)

ou un dérivé de ce composé avec un acide de formule III

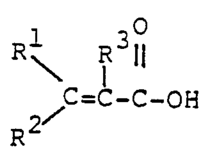

(III)

ou un dérivé réactif de cet acide, ou bien

b) on fait réagir un dérivé réactif d'un acide sulfonique de formule IV

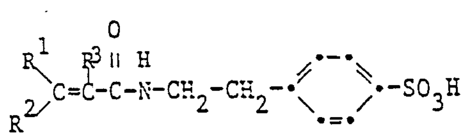

(IV)

avec un composé de formule V

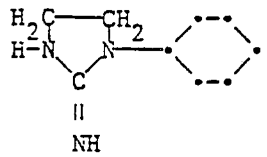

(V)

ou un dérivé de ce composé, ou bien

c) on cyclise un composé de formule VI

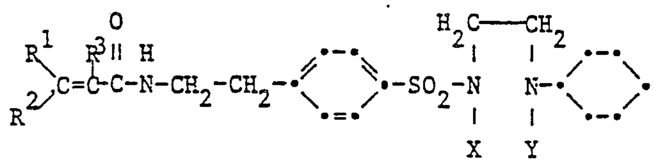

(VI)

dans laquelle l'un des symboles X et Y représente le groupe cyano et l'autre l'hydrogène, et, si on le désire, on convertit un sel obtenu conformément à l'invention en le composé libre ou en un autre sel et/ou un composé libre obtenu conformément à l'invention en un sel et/ou, si on le désire, on sépare un mélange d'isomères en les isomères individuels.

14. Procédé de préparation des composés selon la revendication 1, dans lesquels $R_0^1$ représente un groupe alkyle en C 2 ou C 3, caractérisé en ce que l'on isomérise un composé de formule VII

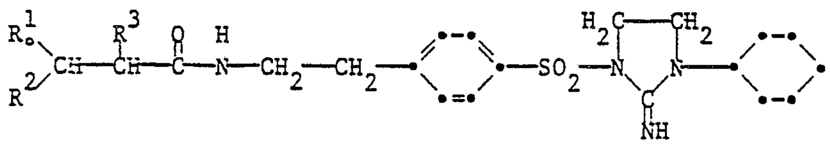

(VII)

dans laquelle $R_0^1$ représente un groupe 1-alcényle en C 2-C 3, ou un dérivé de ce composé, et, si on le désire, on convertit un sel obtenu conformément à l'invention en le composé libre ou en un autre sel et/ou un composé libre obtenu conformément à l'invention en un sel et/ou, si on le désire, on sépare un mélange d'isomères en les isomères individuels.

15. Procédé selon la revendication 13, caractérisé en ce que l'on forme in situ le produit de départ de formule VI de la variante opératoire c)

ca) en faisant réagir un composé de formule VIa

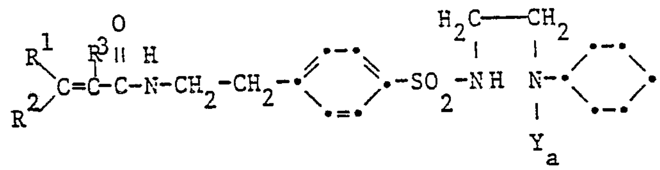

(VIa)

dans laquelle $Y_a$ représente l'hydrogène ou un substituant éliminable dans les conditions de la réaction, avec un dérivé réactif de l'acide cyanique, ou bien

cb) en faisant réagir un composé de formule VIb

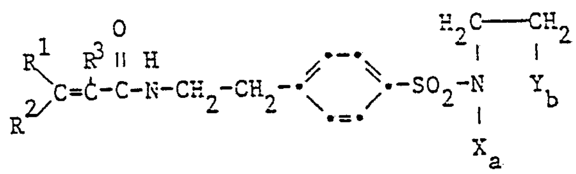

(VIb)

dans laquelle $X_a$ représente l'hydrogène et $Y_b$ un groupe hydroxy estérifié réactif, ou bien dans laquelle $X_a$ et $Y_b$ forment ensemble une liaison, avec le N-cyclohexylcyanamide ou un dérivé de ce composé, ou bien

cc) en faisant réagir un composé de formule VIc

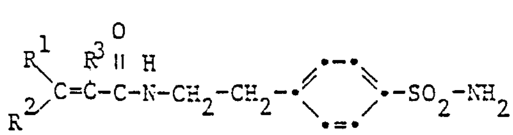

(VIc)

avec un N-cyclohexyl-N-(2-hydroxyéthyl)-cyanamide estérifié réactif ou un dérivé d'un tel composé, ou bien

cd) en cyclisant un sel formé par addition et répondant à la formule VId

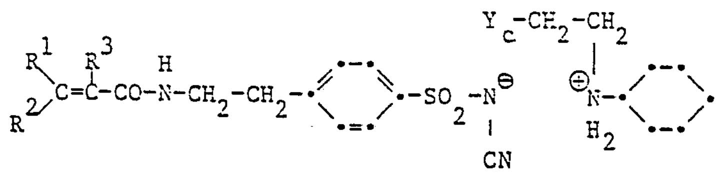

(VId)

dans laquelle $Y_c$ représente un groupe hydroxy estérifié réactif.

**Revendications** (pour l'Etat contractant AT)

1. Procédé de préparation d'un composé de formule I

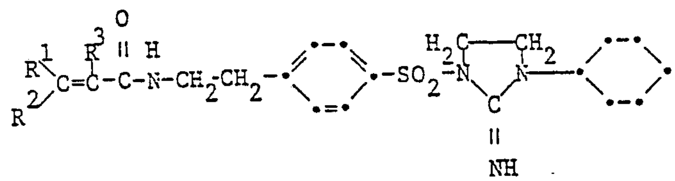

(I)

dans laquelle $R^1$ représente un groupe alkyle en C 1-C 3 et $R^2$ et $R^3$ représentent chacun, indépendamment l'un de l'autre, l'hydrogène ou un groupe alkyle en C 1-C 3, et des sels de ces composés, caractérisé en ce que

a) on fait réagir un composé de formule II

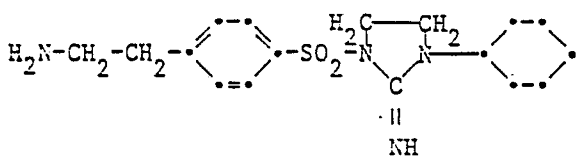

(II)

ou un dérivé de ce composé avec un acide de formule III

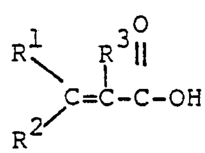

(III)

ou un dérivé réactif de cet acide, ou bien

b) on fait réagir un dérivé réactif d'un acide sulfonique de formule IV

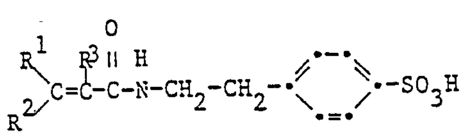

(IV)

avec un composé de formule V

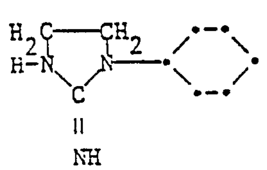

(V)

ou un dérivé de ce composé, ou bien

c) on cyclise un composé de formule VI

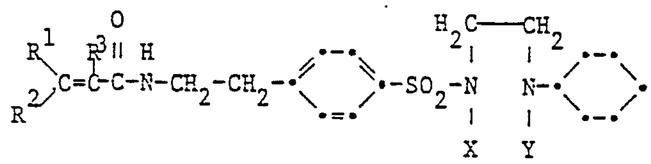

(VI)

dans laquelle l'un des symboles X et Y représente le groupe cyano et l'autre l'hydrogène, et, si on le désire, on convertit un sel obtenu conformément à l'invention en le composé libre ou en un autre sel et/ou un composé libre obtenu conformément à l'invention en un sel et/ou, si on le désire, on sépare un mélange d'isomères en les isomères individuels.

2. Procédé de préparation des composés selon la revendication 1, dans laquelle $R_0^1$ représente un groupe alkyle en C 2 ou C 3, caractérisé en ce que l'on isomérise un composé de formule VII

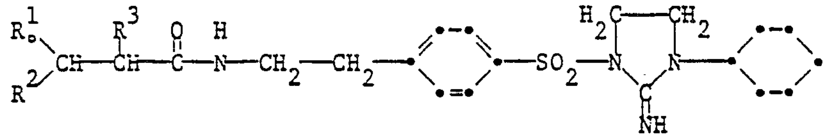

(VII)

dans laquelle $R_0^1$ représente un groupe 1-alcényle en C 2-C 3, ou un dérivé de ce composé, et, si on le désire, on convertit un sel obtenu conformément à l'invention en le composé libre ou en un autre sel et/ou un composé libre obtenu conformément à l'invention en un sel et/ou, si on le désire, on sépare un mélange d'isomères en les isomères individuels.

3. Procédé selon la revendication 1, caractérisé en ce que l'on forme in situ le produit de départ de formule VI de la variante opératoire c)

ca) en faisant réagir un composé de formule VIa

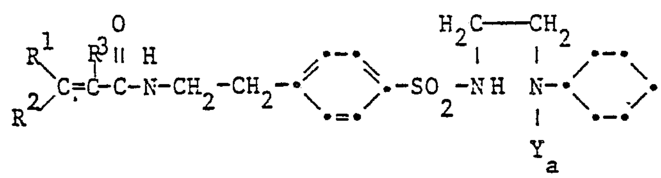

(VIa)

dans laquelle $Y_a$ représente l'hydrogène ou un substituant éliminable dans les conditions de la réaction, avec un dérivé réactif de l'acide cyanique, ou bien

cb) en faisant réagir un composé de formule VIb

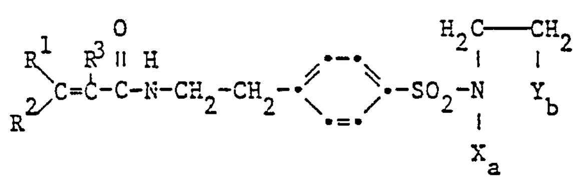

(VIb)

dans laquelle $X_a$ représente l'hydrogène et $Y_b$ un groupe hydroxy estérifié réactif, ou bien dans laquelle $X_a$ et $Y_b$ forment ensemble une liaison, avec le N-cyclohexylcyanamide ou un dérivé de ce composé, ou bien

cc) en faisant réagir un composé de formule VIc

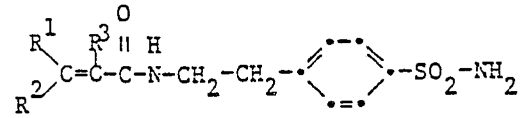

(VIc)

avec un N-cyclohexyl-N-(2-hydroxyéthyl)-cyanamide estérifié réactif ou un dérivé d'un tel composé, ou bien

cd) en cyclisant un sel formé par addition et répondant à la formule VId

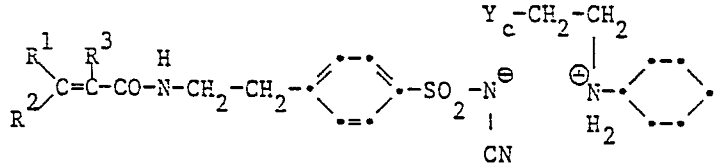

(VId)

dans laquelle $Y_c$ représente un groupe hydroxy estérifié réactif.

4. Procédé selon la revendication 1, caractérisé en ce que l'on prépare des composés de formule I dans laquelle $R^1$ représente un groupe alkyle en C 1-C 3, $R^2$ et $R^3$ représentent l'hydrogène ou un groupe méthyle, ou leurs sels.

5. Procédé selon la revendication 1, caractérisé en ce que l'on prépare la 1-{4-[2-(crotonylamino)-éthyl]-phénylsulfonyl}-3-cyclohexyl-2-imino-imidazolidine.

6. Procédé selon la revendication 1, caractérisé en ce que l'on prépare la 3-cyclohexyl-2-imino-1-{4-[2-(isocrotonylamino)-éthyl]-phénylsulfonyl}-imidazolidine.

7. Procédé selon la revendication 1, caractérisé en ce que l'on prépare la 3-cyclohexyl-2-imino-1-{4-[2-(2,3-diméthylacryl-amino)-éthyl]-phénylsulfonyl}-imidazolidine (trans).

8. Procédé selon la revendication 1, caractérisé en ce que l'on prépare la 3-cyclohexyl-2-imino-1-{4-[2-(4-méthyl-2-penténoyl-amino)-éthyl]-phénylsulfonyl}-2-imino-3-cyclohexyl-imidazolidine (trans).

9. Procédé selon la revendication 1, caractérisé en ce que l'on prépare la 3-cyclohexyl-2-imino-1-{4-[2-(2-hexénoyl-amino)-éthyl]-phénylsulfonyl}-imidazolidine (trans).

10. Procédé de préparation d'une composition pharmaceutique caractérisé en ce que l'on combine une substance active selon les revendications 1 à 8, obtenue conformément à l'invention, avec un véhicule pharmaceutique.